# EUROPEAN PATENT SPECIFICATION

(11) **EP 3 929 299 B1**
(45) Date of publication and mention of the grant of the patent: **10.08.2022**
(21) Application number: 20182382.0
(22) Date of filing: 25.06.2020
(51) Int. Cl.: C12P 7/18, C12N 1/14, C12N 15/09, C12N 9/04

(54) **ERYTHRITOL PRODUCING SAPROTROPH**
ERYTHRITOL PRODUZIERENDER SAPROTROPH
SAPROTROPHE PRODUCTRICE D'ÉRYTHRITOL

(43) Date of publication of application: 29.12.2021
(73) Proprietor: Conzil Estate GmbH, 78351 Bodman-Ludwigshafen (DE)
(72) Inventor: Mach, Robert, 1060 Vienna (AT); Mach-Aigner, Astrid, 1060 Vienna (AT); Regnat, Katharina, 1030 Vienna (AT)
(74) Representative: Schrell, Andreas

(56) References cited:
- EP-A1- 2 436 772
- ISHIZUKA H ET AL: "PURIFICATION AND SOME PROPERTIES OF AN ERYTHROSE REDUCTASE FROM AN AUREOBASIDIUM SP. MUTANT", BIOSCIENCE BIOTECHNOLOGY BIOCHEMISTRY, JAPAN SOCIETY FOR BIOSCIENCE, BIOTECHNOLOGY AND AGROCHEMISTRY, JP, vol. 56, no. 6, 1 January 1992 (1992-01-01), pages 941-945, XP001073699, ISSN: 0916-8451
- VELEZ ET AL: "Mannitol metabolism in the phytopathogenic fungus Alternaria alternata", FUNGAL GENETICS AND BIOLOGY, SAN DIEGO, CA, US, vol. 44, no. 4, 2 March 2007 (2007-03-02), pages 258-268, XP005907328, ISSN: 1087-1845, DOI: 10.1016/J.FGB.2006.09.008
- HEE-JUNG MOON ET AL: "Biotechnological production of erythritol and its applications", APPLIED MICROBIOLOGY AND BIOTECHNOLOGY, SPRINGER, BERLIN, DE, vol. 86, no. 4, 26 February 2010 (2010-02-26), pages 1017-1025, XP019800001, ISSN: 1432-0614

### SEQUENCE LISTING

<110> Conzil Estate GmbH
<120> Erythritol producing saprotroph
<130> 208477
<160> 25
<170> PatentIn version 3.5
<210> 1
   <211> 2010
   <212> DNA
   <213> Saccharomyces cerevisiae
<400> 1
   atgagcaatc cccaaaaggc actgaacgac ttcctcagct ccgagagcgt ccacacccac 60
   gactcctcac gtaagcagtc caacaagcaa tcctcggacg agggccgtag ctcgagccag 120
   ccctcccacc atcactccgg aggaactaat aacaataaca acaataacaa caacaacaac 180
   aacagcaaca acaacaacaa cggtaacgat ggcggcaacg acgacgacta tgactacgag 240
   atgcaggact accgtccctc cccacagtct gctaggccta ctcccaccta cgtgccccaa 300
   tacagtgtcg agtcgggcac cgcattcccc attcaagagg tcattccctc ggcctacatt 360
   aacacccaag acatcaacca caaggacaac ggcccccctt ccgcatcgtc aaaccgcgct 420
   ttccgccccc gcggccagac gaccgtcagc gccaacgtcc tgaacataga ggacttctac 480
   aagaatgcgg acgatgcaca cacaattcca gagtcgcatc tgagccggcg tcgctcccga 540
   tcacgggcca cgtcaaatgc aggccactcc gccaacaccg gcgccaccaa tggtcgtact 600
   acgggcgccc agaccaacat ggagagcaac gagtccccca ggaacgtgcc gataatggtc 660
   aagcctaaga ccctctacca gaacccgcag acccccacag ttctcccctc aacttaccac 720
   ccgattaaca agtggagctc cgttaagaat acctatttaa aagagttcct cgccgagttt 780
   atgggtacga tggtcatgat catcttcggc agcgccgtcg tatgccaggt caacgtcgcc 840
   ggcaagatcc agcaggacaa cttcaacgta gccctcgaca acctcaatgt cacgggctct 900
   tccgccgaga caatcgacgc catgaagagc ctgactagcc tagtctcctc tgtcgccggt 960
   ggcaccttcg atgacgttgc cctcgggtgg gccgctgccg tggtaatggg ctacttttgt 1020
   gcaggtggct cggcaatctc tggagcccac ctgaacccat caatcaccct cgctaacctc 1080
   gtttatcgag gctttcccct gaagaaggtc ccatattact tcgcaggcca gctgattggt 1140
   gctttcacgg gagccctcat tctgttcatt tggtacaaga gggtccttca ggaggcatac 1200
   tcggactggt ggatgaacga atccgtcgct ggcatgtttt gtgtcttccc aaagccctac 1260
   ctgagctctg ggcggcagtt ttttagcgag ttcctctgcg gcgccatgct ccaggccggg 1320
   acattcgccc tgacggatcc ctacacgtgc ttgagctcag atgtatttcc cctgatgatg 1380
   ttcattctca ttttcatcat taacgcttcg atggcatatc agacaggtac tgcgatgaac 1440
   ctggcacggg atctgggccc ccgcctcgcc ctctacgcag tgggcttcga ccacaagatg 1500
   ctctgggtcc accaccacca cttcttttgg gtgccgatgg tagggccgtt tattggtgcc 1560
   ctgatggggg gcctcgtcta cgacgtctgt atctaccaag gccacgaatc accggttaac 1620
   tggtctctcc ccgtgtacaa ggaaatgatt atgcgcgcat ggtttaggcg ccccggatgg 1680
   aagaagcgga accgagctag gaggacttct gacctctccg acttctctta caataatgac 1740
   gacgacgagg aattcggcga acgcatggcg cttcagaaga cgaagacgaa gtctagcatt 1800
   tccgacaacg agaacgaggc cggcgagaag aaggttcagt tcaagtccgt tcagcgaggg 1860
   aagcggacct tcggcggcat cccgacaatt cttgaggagg aggatagcat cgagactgcc 1920
   agcctcggcg ccacgacaac ggattctatt ggtctctctg acactagctc tgaggacagc 1980
   cactacggca acgcgaagaa agtcacctga 2010
<210> 2
   <211> 669
   <212> PRT
   <213> Saccharomyces cerevisiae
<400> 2
   Met Ser Asn Pro Gln Lys Ala Leu Asn Asp Phe Leu Ser Ser Glu Ser
   1 5 10 15
   Val His Thr His Asp Ser Ser Arg Lys Gln Ser Asn Lys Gln Ser Ser
   20 25 30
   Asp Glu Gly Arg Ser Ser Ser Gln Pro Ser His His His Ser Gly Gly
   35 40 45
   Thr Asn Asn Asn Asn Asn Asn Asn Asn Asn Asn Asn Asn Ser Asn Asn
   50 55 60
   Asn Asn Asn Gly Asn Asp Gly Gly Asn Asp Asp Asp Tyr Asp Tyr Glu
   65 70 75 80
   Met Gln Asp Tyr Arg Pro Ser Pro Gln Ser Ala Arg Pro Thr Pro Thr
   85 90 95
   Tyr Val Pro Gln Tyr Ser Val Glu Ser Gly Thr Ala Phe Pro Ile Gln
   100 105 110
   Glu Val Ile Pro Ser Ala Tyr Ile Asn Thr Gln Asp Ile Asn His Lys
   115 120 125
   Asp Asn Gly Pro Pro Ser Ala Ser Ser Asn Arg Ala Phe Arg Pro Arg
       130                 135                 140
   Gly Gln Thr Thr Val Ser Ala Asn Val Leu Asn Ile Glu Asp Phe Tyr
   145                 150                 155                 160
   Lys Asn Ala Asp Asp Ala His Thr Ile Pro Glu Ser His Leu Ser Arg
                   165                 170                 175
   Arg Arg Ser Arg Ser Arg Ala Thr Ser Asn Ala Gly His Ser Ala Asn
               180                 185                 190
   Thr Gly Ala Thr Asn Gly Arg Thr Thr Gly Ala Gln Thr Asn Met Glu
           195                 200                 205
   Ser Asn Glu Ser Pro Arg Asn Val Pro Ile Met Val Lys Pro Lys Thr
       210                 215                 220
   Leu Tyr Gln Asn Pro Gln Thr Pro Thr Val Leu Pro Ser Thr Tyr His
   225                 230                 235                 240
   Pro Ile Asn Lys Trp Ser Ser Val Lys Asn Thr Tyr Leu Lys Glu Phe
                   245                 250                 255
   Leu Ala Glu Phe Met Gly Thr Met Val Met Ile Ile Phe Gly Ser Ala
               260                 265                 270
   Val Val Cys Gln Val Asn Val Ala Gly Lys Ile Gln Gln Asp Asn Phe
           275                 280                 285
   Asn Val Ala Leu Asp Asn Leu Asn Val Thr Gly Ser Ser Ala Glu Thr
       290                 295                 300
   Ile Asp Ala Met Lys Ser Leu Thr Ser Leu Val Ser Ser Val Ala Gly
   305                 310                 315                 320
   Gly Thr Phe Asp Asp Val Ala Leu Gly Trp Ala Ala Ala Val Val Met
                   325                 330                 335
   Gly Tyr Phe Cys Ala Gly Gly Ser Ala Ile Ser Gly Ala His Leu Asn
               340                 345                 350
   Pro Ser Ile Thr Leu Ala Asn Leu Val Tyr Arg Gly Phe Pro Leu Lys
           355                 360                 365
   Lys Val Pro Tyr Tyr Phe Ala Gly Gln Leu Ile Gly Ala Phe Thr Gly
       370                 375                 380
   Ala Leu Ile Leu Phe Ile Trp Tyr Lys Arg Val Leu Gln Glu Ala Tyr
   385                 390                 395                 400
   Ser Asp Trp Trp Met Asn Glu Ser Val Ala Gly Met Phe Cys Val Phe
                   405                 410                 415
   Pro Lys Pro Tyr Leu Ser Ser Gly Arg Gln Phe Phe Ser Glu Phe Leu
               420                 425                 430
   Cys Gly Ala Met Leu Gln Ala Gly Thr Phe Ala Leu Thr Asp Pro Tyr
           435                 440                 445
   Thr Cys Leu Ser Ser Asp Val Phe Pro Leu Met Met Phe Ile Leu Ile
       450                 455                 460
   Phe Ile Ile Asn Ala Ser Met Ala Tyr Gln Thr Gly Thr Ala Met Asn
   465                 470                 475                 480
   Leu Ala Arg Asp Leu Gly Pro Arg Leu Ala Leu Tyr Ala Val Gly Phe
                   485                 490                 495
   Asp His Lys Met Leu Trp Val His His His His Phe Phe Trp Val Pro
               500                 505                 510
   Met Val Gly Pro Phe Ile Gly Ala Leu Met Gly Gly Leu Val Tyr Asp
           515                 520                 525
   Val Cys Ile Tyr Gln Gly His Glu Ser Pro Val Asn Trp Ser Leu Pro
       530                 535                 540
   Val Tyr Lys Glu Met Ile Met Arg Ala Trp Phe Arg Arg Pro Gly Trp
   545                 550                 555                 560
   Lys Lys Arg Asn Arg Ala Arg Arg Thr Ser Asp Leu Ser Asp Phe Ser
                   565                 570                 575
   Tyr Asn Asn Asp Asp Asp Glu Glu Phe Gly Glu Arg Met Ala Leu Gln
               580                 585                 590
   Lys Thr Lys Thr Lys Ser Ser Ile Ser Asp Asn Glu Asn Glu Ala Gly
           595                 600                 605
   Glu Lys Lys Val Gln Phe Lys Ser Val Gln Arg Gly Lys Arg Thr Phe
       610                 615                 620
   Gly Gly Ile Pro Thr Ile Leu Glu Glu Glu Asp Ser Ile Glu Thr Ala
   625                 630                 635                 640
   Ser Leu Gly Ala Thr Thr Thr Asp Ser Ile Gly Leu Ser Asp Thr Ser
   645 650 655
   Ser Glu Asp Ser His Tyr Gly Asn Ala Lys Lys Val Thr
   660 665
<210> 3
   <211> 996
   <212> DNA
   <213> Trichoderma reesei
<400> 3
   atgtcttccg gaaggaccgt caccctcaac accggctaca agatccccca gatcggctac      60
   ggcacctggc aggccgctcc cggcgaggtc ggcgctggtg tctttgaggc cctcaaggtt     120
   ggctaccgcc acctcgacct ggccaaggtc tacggcaacc agaaggaggt tggtgagggc     180
   atcaagaagg ctcttgctga ggtccccggc ctgaagcgcg aggatatttt catcacctcc     240
   aagctgtgga acaactccca caagcccgag gacgtcgagc ccgctctcga cgacaccctg     300
   gccgagcttg gcctcgacta ccttgacctc tacctcatcc actggcccgt tgcctttgct     360
   cccggcgccg acctcttccc caagtccgag gacggctccg aggtgcagct caaccagaat     420
   gtgtccattg tccagacctg gaaggccatg accgagctgc ccaagtccaa ggtccgctcc     480
   gtcggtgtct ccaactttac catagagcac ctcgacgccg tcatcgaggc caccggcgtc     540
   gtccccgccg tcaaccagat cgagcgccac ccccgcctcc ccaaccagcc tctgatcgac     600
   tactgcgcca agaagggcat catcatcacc gcctactccg cctttggcaa caacacaaag     660
   ggcctgcccc tgctcgtcag ctccgacgag gtcaaggccg tcgccgacaa cctgtccaag     720
   aagcagggca agaccgtcac tcccgcccag gtcatcctcg cctggtccca gattggtggc     780
   cacaccgtca ttcccaagtc cgtcaccaag gcgcgcattg cggagaactt ccaggaggtt     840
   gagctggatg acgaggccat tgctgcgctg aacaagttgg gcgagaagcc tcagcggttc     900
   aacattcctt acacctacaa gcctaggtgg aacattaacc tgttcaacac cgaggaggag     960
   aaggccgctg cccacactgc tgtcatcaag ctgtaa                               996
<210> 4
   <211> 331
   <212> PRT
   <213> Trichoderma reesei
<400> 4
   Met Ser Ser Gly Arg Thr Val Thr Leu Asn Thr Gly Tyr Lys Ile Pro
   1               5                   10                  15
   Gln Ile Gly Tyr Gly Thr Trp Gln Ala Ala Pro Gly Glu Val Gly Ala
   20 25 30
   Gly Val Phe Glu Ala Leu Lys Val Gly Tyr Arg His Leu Asp Leu Ala
   35 40 45
   Lys Val Tyr Gly Asn Gln Lys Glu Val Gly Glu Gly Ile Lys Lys Ala
   50 55 60
   Leu Ala Glu Val Pro Gly Leu Lys Arg Glu Asp Ile Phe Ile Thr Ser
   65 70 75 80
   Lys Leu Trp Asn Asn Ser His Lys Pro Glu Asp Val Glu Pro Ala Leu
   85 90 95
   Asp Asp Thr Leu Ala Glu Leu Gly Leu Asp Tyr Leu Asp Leu Tyr Leu
   100 105 110
   Ile His Trp Pro Val Ala Phe Ala Pro Gly Ala Asp Leu Phe Pro Lys
   115 120 125
   Ser Glu Asp Gly Ser Glu Val Gln Leu Asn Gln Asn Val Ser Ile Val
   130 135 140
   Gln Thr Trp Lys Ala Met Thr Glu Leu Pro Lys Ser Lys Val Arg Ser
   145 150 155 160
   Val Gly Val Ser Asn Phe Thr Ile Glu His Leu Asp Ala Val Ile Glu
   165 170 175
   Ala Thr Gly Val Val Pro Ala Val Asn Gln Ile Glu Arg His Pro Arg
   180 185 190
   Leu Pro Asn Gln Pro Leu Ile Asp Tyr Cys Ala Lys Lys Gly Ile Ile
   195 200 205
   Ile Thr Ala Tyr Ser Ala Phe Gly Asn Asn Thr Lys Gly Leu Pro Leu
   210 215 220
   Leu Val Ser Ser Asp Glu Val Lys Ala Val Ala Asp Asn Leu Ser Lys
   225 230 235 240
   Lys Gln Gly Lys Thr Val Thr Pro Ala Gln Val Ile Leu Ala Trp Ser
   245 250 255
   Gln Ile Gly Gly His Thr Val Ile Pro Lys Ser Val Thr Lys Ala Arg
   260 265 270
   Ile Ala Glu Asn Phe Gln Glu Val Glu Leu Asp Asp Glu Ala Ile Ala
           275                 280                 285
   Ala Leu Asn Lys Leu Gly Glu Lys Pro Gln Arg Phe Asn Ile Pro Tyr
       290                 295                 300
   Thr Tyr Lys Pro Arg Trp Asn Ile Asn Leu Phe Asn Thr Glu Glu Glu
   305                 310                 315                 320
   Lys Ala Ala Ala His Thr Ala Val Ile Lys Leu
                   325                 330
<210> 5
   <211> 2055
   <212> DNA
   <213> Trichoderma reesei
<400> 5
   atgggctaca cagaggttga ccagaaggcc atcaacacca tccgcctgct cgcggtcgat      60
   gccaccttca agagcaactc gggccacccg ggtgccccca tgggcatggc tcccgttgcc     120
   cacgtccttt gggacaagtt catgaggttc aaccccaaga accccaagtg gctgaaccgt     180
   gaccgattcg ttctctccaa cggccacggc tgcatgctcc agtatgctct gcttcacctg     240
   ttcggctacg acctgaccat cgatgacctc aaggccttcc gaaccgtcga cagcaagact     300
   cctggtcacc ccgaggccca cgatactccc ggtgtcgagg tcaccaccgg ccccctgggt     360
   cagggtatct ccaatgccgt cggtctggcc atggcccagg cccacaccgc cgccaccttc     420
   aacaagcccg gcttcaccct gtgtgacaac tacacctact gcttcctcgg cgatggctgc     480
   ttgatggagg gtgtctctgc cgaggcctcg tccctggccg gccacctcca gctcggcaac     540
   ctcatctgca tctacgatga caaccacatc tccattgacg gcgacaccaa ctgtgccttc     600
   accgaggacg tggccaagcg ctacgaggcc tacggctggc acgtcgaggt cgtcgagaac     660
   ggcgaccacg acctcgaggc catggaggcc gccatcaaga agtgccagga ggtcaaggac     720
   aagccctcaa tcatcaagct gcgcaccacc attggcttcg gctctctcca ggagggcacc     780
   cacggagtcc acggctctcc cctcaaggcc gacgacatca agcagctcaa gcagaagttt     840
   ggcttcaacc ccgacgagag cttcgtcgtc cccaaggagg tctccgacct ctacgcccgc     900
   cactcagccg agggcgccgc cgccgaggag gagtggaaca agctgttcgc caagtacgcc     960
   gagaagtacc ccgccgaggc tgctgacctc cagcgccgcc tcaagggcga gctgcccgag    1020
   ggctgggaga agaacctgcc cgtctactcg cccagcgatg ctgccattgc cagccgaaag    1080
   ctgtccgaga cggtcctcac caagatccac gacgtcctgc ccgagctcca gggtggttcc    1140
   gccgatctga ctggttccaa cctgacccgc tggaaggacg ctgttgactt ccagcccccc    1200
   gccaccggcc tcggtgacta ctccggacga tacatccgct acggtgtccg cgagcacggc    1260
   atgggcgcca tcatgaacgg cctggccgcc tacggcacca tcatccccta cggcggtacc    1320
   ttcctcaact tcgtctccta cgccgccggt gccgtccgcc tgtccgctct gtcccgcgtc    1380
   cgcgtcatct gggtcgccac ccacgactcc attggcctgg gcgaggacgg tcctactcac    1440
   cagcctatcg agactctggc tcacttccgc gccctgccca actgcatggt gtggcgcccc    1500
   gccgacggca acgagaccag cgctgcctac tacattgccc tgacctccaa gcacaccccc    1560
   agcatcattg ccctgtctcg ccagaacctg ccccagctcg agggctccgt catcgagaag    1620
   gccatcaagg gaggatacgt cctgcaggag gtcgagggtg cccagatcac cctggtctcc    1680
   accggttccg aggtttccat cgccgtggat gccgccaagt acctcctgga gaagcacaac    1740
   gtcaaggccc gcgtcgtctc catgccctgc ttcgaggttt tcgatgctca gcccaaggag    1800
   tacagactct ccgtcctgcc cgacggcatc ccctctctgt ccattgaggt gatgagcacc    1860
   atgggctggg agcgatacac tcacgagcag tttggtctca accgattcgg tgcctctggc    1920
   gcctacaagg atgtctacaa gaagttcgag ttcacccccg agggcatcac caagcgcgct    1980
   cttctcacca ttgacttctg gaaggacgtc cccaacgtcc gctctcccat caaccgtgcc    2040
   ttccagcagc tcatc                                                    2055
<210> 6
   <211> 685
   <212> PRT
   <213> Trichoderma reesei
<400> 6
   Met Gly Tyr Thr Glu Val Asp Gln Lys Ala Ile Asn Thr Ile Arg Leu
   1               5                   10                  15
   Leu Ala Val Asp Ala Thr Phe Lys Ser Asn Ser Gly His Pro Gly Ala
               20                  25                  30
   Pro Met Gly Met Ala Pro Val Ala His Val Leu Trp Asp Lys Phe Met
           35                  40                  45
   Arg Phe Asn Pro Lys Asn Pro Lys Trp Leu Asn Arg Asp Arg Phe Val
       50                  55                  60
   Leu Ser Asn Gly His Gly Cys Met Leu Gln Tyr Ala Leu Leu His Leu
   65                  70                  75                  80
   Phe Gly Tyr Asp Leu Thr Ile Asp Asp Leu Lys Ala Phe Arg Thr Val
                   85                  90                  95
   Asp Ser Lys Thr Pro Gly His Pro Glu Ala His Asp Thr Pro Gly Val
               100                 105                 110
   Glu Val Thr Thr Gly Pro Leu Gly Gln Gly Ile Ser Asn Ala Val Gly
   115 120 125
   Leu Ala Met Ala Gln Ala His Thr Ala Ala Thr Phe Asn Lys Pro Gly
   130 135 140
   Phe Thr Leu Cys Asp Asn Tyr Thr Tyr Cys Phe Leu Gly Asp Gly Cys
   145 150 155 160
   Leu Met Glu Gly Val Ser Ala Glu Ala Ser Ser Leu Ala Gly His Leu
   165 170 175
   Gln Leu Gly Asn Leu Ile Cys Ile Tyr Asp Asp Asn His Ile Ser Ile
   180 185 190
   Asp Gly Asp Thr Asn Cys Ala Phe Thr Glu Asp Val Ala Lys Arg Tyr
   195 200 205
   Glu Ala Tyr Gly Trp His Val Glu Val Val Glu Asn Gly Asp His Asp
   210 215 220
   Leu Glu Ala Met Glu Ala Ala Ile Lys Lys Cys Gln Glu Val Lys Asp
   225 230 235 240
   Lys Pro Ser Ile Ile Lys Leu Arg Thr Thr Ile Gly Phe Gly Ser Leu
   245 250 255
   Gln Glu Gly Thr His Gly Val His Gly Ser Pro Leu Lys Ala Asp Asp
   260 265 270
   Ile Lys Gln Leu Lys Gln Lys Phe Gly Phe Asn Pro Asp Glu Ser Phe
   275 280 285
   Val Val Pro Lys Glu Val Ser Asp Leu Tyr Ala Arg His Ser Ala Glu
   290 295 300
   Gly Ala Ala Ala Glu Glu Glu Trp Asn Lys Leu Phe Ala Lys Tyr Ala
   305 310 315 320
   Glu Lys Tyr Pro Ala Glu Ala Ala Asp Leu Gln Arg Arg Leu Lys Gly
   325 330 335
   Glu Leu Pro Glu Gly Trp Glu Lys Asn Leu Pro Val Tyr Ser Pro Ser
   340 345 350
   Asp Ala Ala Ile Ala Ser Arg Lys Leu Ser Glu Thr Val Leu Thr Lys
   355 360 365
   Ile His Asp Val Leu Pro Glu Leu Gln Gly Gly Ser Ala Asp Leu Thr
   370 375 380
   Gly Ser Asn Leu Thr Arg Trp Lys Asp Ala Val Asp Phe Gln Pro Pro
   385 390 395 400
   Ala Thr Gly Leu Gly Asp Tyr Ser Gly Arg Tyr Ile Arg Tyr Gly Val
   405 410 415
   Arg Glu His Gly Met Gly Ala Ile Met Asn Gly Leu Ala Ala Tyr Gly
   420 425 430
   Thr Ile Ile Pro Tyr Gly Gly Thr Phe Leu Asn Phe Val Ser Tyr Ala
   435 440 445
   Ala Gly Ala Val Arg Leu Ser Ala Leu Ser Arg Val Arg Val Ile Trp
   450 455 460
   Val Ala Thr His Asp Ser Ile Gly Leu Gly Glu Asp Gly Pro Thr His
   465 470 475 480
   Gln Pro Ile Glu Thr Leu Ala His Phe Arg Ala Leu Pro Asn Cys Met
   485 490 495
   Val Trp Arg Pro Ala Asp Gly Asn Glu Thr Ser Ala Ala Tyr Tyr Ile
   500 505 510
   Ala Leu Thr Ser Lys His Thr Pro Ser Ile Ile Ala Leu Ser Arg Gln
   515 520 525
   Asn Leu Pro Gln Leu Glu Gly Ser Val Ile Glu Lys Ala Ile Lys Gly
   530 535 540
   Gly Tyr Val Leu Gln Glu Val Glu Gly Ala Gln Ile Thr Leu Val Ser
   545 550 555 560
   Thr Gly Ser Glu Val Ser Ile Ala Val Asp Ala Ala Lys Tyr Leu Leu
   565 570 575
   Glu Lys His Asn Val Lys Ala Arg Val Val Ser Met Pro Cys Phe Glu
   580 585 590
   Val Phe Asp Ala Gln Pro Lys Glu Tyr Arg Leu Ser Val Leu Pro Asp
   595 600 605
   Gly Ile Pro Ser Leu Ser Ile Glu Val Met Ser Thr Met Gly Trp Glu
       610                 615                 620
   Arg Tyr Thr His Glu Gln Phe Gly Leu Asn Arg Phe Gly Ala Ser Gly
   625                 630                 635                 640
   Ala Tyr Lys Asp Val Tyr Lys Lys Phe Glu Phe Thr Pro Glu Gly Ile
                   645                 650                 655
   Thr Lys Arg Ala Leu Leu Thr Ile Asp Phe Trp Lys Asp Val Pro Asn
               660                 665                 670
   Val Arg Ser Pro Ile Asn Arg Ala Phe Gln Gln Leu Ile
           675                 680                 685
<210> 7
   <211> 975
   <212> DNA
   <213> Trichoderma reesei
<400> 7
   atgagctcct ctcttgacca gctcaaggcc actggcaccg ttgtcgtctc tgacactggt      60
   gactttgctg ccatcgccaa gtacaagccc caggatgcca ccaccaaccc ctccctcatc     120
   ctggccgcct ccaagaagcc cgagtacgcc aagctgatag acgtcgccat cgactacgcc     180
   aagcagaagg gcggcgacat tgagcagcag gtcgacgatg ccctcgaccg cctgctggtc     240
   gagttcggca aggagattct caagattgtt cccggcaagg tctccaccga ggtcgacgcc     300
   cggttctcct ttgacaccaa ggcctctgtc gacaaggccc tccacattat cgagctctac     360
   aaggagctcg gcatccccaa ggagcgcgtc ctcatcaaga tcgcctccac ctgggagggc     420
   atcaaggccg ccgagatcct gcagcgcgac cacggcatca actgcaacct gacgctcatg     480
   ttctccctgc cccaggccat cggcgccgcc gaggccggcg ccttcctcat ctcccccttc     540
   gttggccgca tcctcgactg gttcaaggcg agcaccaaga aggactactc caaggaggag     600
   gaccccggtg tcgcctccgt caagagcatc ttcaactact acaagaagtt tggctacaag     660
   accattgtca tgggtgcctc gttccgcaac acgggcgaga ttaccgagct cgctggctgc     720
   gactacctga ccatttctcc caacctgctc gaggagctcc tcaactccac cgagcccgtc     780
   cccaagaagc tcgacgccgc cgccgctgcc tcccttaaca ttgagaagaa gtcgtacatc     840
   aacgacgagt ccctgttccg cttcgacttc aacgaggacc agatggccgt cgagaagctg     900
   cgcgagggca tcagcaagtt tgccgccgac gccgtcaccc tcaaggacat tctgaggcag     960
   aagctgtctg cttaa                                                      975
<210> 8
   <211> 324
   <212> PRT
   <213> Trichoderma reesei
<400> 8
   Met Ser Ser Ser Leu Asp Gln Leu Lys Ala Thr Gly Thr Val Val Val
   1               5                   10                  15
   Ser Asp Thr Gly Asp Phe Ala Ala Ile Ala Lys Tyr Lys Pro Gln Asp
               20                  25                  30
   Ala Thr Thr Asn Pro Ser Leu Ile Leu Ala Ala Ser Lys Lys Pro Glu
           35                  40                  45
   Tyr Ala Lys Leu Ile Asp Val Ala Ile Asp Tyr Ala Lys Gln Lys Gly
       50                  55                  60
   Gly Asp Ile Glu Gln Gln Val Asp Asp Ala Leu Asp Arg Leu Leu Val
   65                  70                  75                  80
   Glu Phe Gly Lys Glu Ile Leu Lys Ile Val Pro Gly Lys Val Ser Thr
                   85                  90                  95
   Glu Val Asp Ala Arg Phe Ser Phe Asp Thr Lys Ala Ser Val Asp Lys
               100                 105                 110
   Ala Leu His Ile Ile Glu Leu Tyr Lys Glu Leu Gly Ile Pro Lys Glu
           115                 120                 125
   Arg Val Leu Ile Lys Ile Ala Ser Thr Trp Glu Gly Ile Lys Ala Ala
       130                 135                 140
   Glu Ile Leu Gln Arg Asp His Gly Ile Asn Cys Asn Leu Thr Leu Met
   145                 150                 155                 160
   Phe Ser Leu Pro Gln Ala Ile Gly Ala Ala Glu Ala Gly Ala Phe Leu
                   165                 170                 175
   Ile Ser Pro Phe Val Gly Arg Ile Leu Asp Trp Phe Lys Ala Ser Thr
               180                 185                 190
   Lys Lys Asp Tyr Ser Lys Glu Glu Asp Pro Gly Val Ala Ser Val Lys
           195                 200                 205
   Ser Ile Phe Asn Tyr Tyr Lys Lys Phe Gly Tyr Lys Thr Ile Val Met
       210                 215                 220
   Gly Ala Ser Phe Arg Asn Thr Gly Glu Ile Thr Glu Leu Ala Gly Cys
   225                 230                 235                 240
   Asp Tyr Leu Thr Ile Ser Pro Asn Leu Leu Glu Glu Leu Leu Asn Ser
   245 250 255
   Thr Glu Pro Val Pro Lys Lys Leu Asp Ala Ala Ala Ala Ala Ser Leu
   260 265 270
   Asn Ile Glu Lys Lys Ser Tyr Ile Asn Asp Glu Ser Leu Phe Arg Phe
   275 280 285
   Asp Phe Asn Glu Asp Gln Met Ala Val Glu Lys Leu Arg Glu Gly Ile
   290 295 300
   Ser Lys Phe Ala Ala Asp Ala Val Thr Leu Lys Asp Ile Leu Arg Gln
   305 310 315 320
   Lys Leu Ser Ala
<210> 9
   <211> 9538
   <212> DNA
   <213> artificial sequence
<220>
   <223> pKR_ReAsl1fps1
<400> 9
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtggccgct acagggcgct cccattcgcc attcaggctg cgcaactgtt      180
   gggaagggcg tttcggtgcg ggcctcttcg ctattacgcc agctggcgaa agggggatgt      240
   gctgcaaggc gattaagttg ggtaacgcca gggttttccc agtcacgacg ttgtaaaacg      300
   acggccagtg agcgcgacgt aatacgactc actatagggc gaattgagtg aaggccgtca      360
   aggcctaggc gcgccagcgg ccgcgaaata gaagacgaag acgagaggag gagagggtgc      420
   gagagggtgc gaaactgcgc ccttcagctc gctaattccg cccattacat tcagcgcaca      480
   cttggtggta catacatacc tacatacgta cccgtagttg tgcgaagcga gtcgagccag      540
   cggtcggatg tgccaatatg taccttgtag gtactcatga tagtcagcag gaagccgagg      600
   gatgatagca cgcacagcca gtctggtgtt tgtgctctgg attcagcccc tagattcact      660
   gaccaaaaga agcaagagaa gggaaaggga aaaggaagcc ctaaaaaaga gcagggttgt      720
   ccttcggcac caccccgaaa gcaacagcag tgatggacgg gactacgaca ctgtagtaca      780
   tacgccaacc caaagattcc gagttgcaat cattgccaca aaattgctcg actgtggcgg      840
   gagaggggaa agtctccgcg agggcatctc tgatgagtcc cgtgccacgc cgggtttctt 900
   gccttgaatc accacaaaat gtctcagcgc ggctcatcca tggccgcctt tttgggcgtt 960
   tcgtattact cttgggtttg actgtattat cggtcaagag ccaaaggcca ctcggctttt 1020
   ccgcccttgc ctctcttcca tttccccagg cgctgatagt atgatagttt cccctcattg 1080
   cagctttgga cgtcaatgcc aataactgta ttgtattgac aaatcatttg tccaaagacg 1140
   accgacacct ctgatatcac atacgagtag tagtacatac aatccccccc aaactccgcc 1200
   tttttggcgc gtgtgtgccg cgtagctcgt gcacaatgtc gggccgccgc cgccagcggc 1260
   cacttgctta gcaggaacct agtcagccgt ccttgtgtga ttaatataac ggtgagacta 1320
   gcggccggtc ccttatccag ctgtccacgt tggcctgccc tcagttagcg ctcaactcat 1380
   gccctcactg gcgaggcgag ggcaaggatg gaggggcagc atcgcctgag ttggagcaaa 1440
   gcgccgccat gggagcagcg aaccaacgga gggatgccgt gctttgtcgt ggctgctgtg 1500
   gccaatccgg gcccttggtt ggctcacaga gcgttgctgt gagaccatga gctattattg 1560
   ctaggtacag tatagagaga ggagagagag agagagagtg tgagagggaa aaggtgaggt 1620
   tgaagtgagg tagtaagtga aaaaaaaaaa aaaatcaaca ctgacggctg ccgtctgcca 1680
   ccctcctcca cccagccacc tgcacactca gcgcgcagca tcacctaatc ttggctcgcc 1740
   ttccgcagct caggttgttt tttttttttc tctctcctcg tcgaagccgc ccttgttccc 1800
   ttatttattt ccctctccta ccttgtctgc ctttggtcca tctgcccctt tgtctgcatc 1860
   tcttttgcac gcatcgcctt atcgtcgtct cttttttcac tcacgggagc ttgacgaaga 1920
   cctgactcgt gagcctcacc tgctgatttc tctccccccc tcccgaccgg cttgactttt 1980
   gtttctcctc cagtacctta tcgcgaagcc ggaagaaccc tcttaacctc tagaatgagc 2040
   aatccccaaa aggcactgaa cgacttcctc agctccgaga gcgtccacac ccacgactcc 2100
   tcacgtaagc agtccaacaa gcaatcctcg gacgagggcc gtagctcgag ccagccctcc 2160
   caccatcact ccggaggaac taataacaat aacaacaata acaacaacaa caacaacagc 2220
   aacaacaaca acaacggtaa cgatggcggc aacgacgacg actatgacta cgagatgcag 2280
   gactaccgtc cctccccaca gtctgctagg cctactccca cctacgtgcc ccaatacagt 2340
   gtcgagtcgg gcaccgcatt ccccattcaa gaggtcattc cctcggccta cattaacacc 2400
   caagacatca accacaagga caacggcccc ccttccgcat cgtcaaaccg cgctttccgc 2460
   ccccgcggcc agacgaccgt cagcgccaac gtcctgaaca tagaggactt ctacaagaat 2520
   gcggacgatg cacacacaat tccagagtcg catctgagcc ggcgtcgctc ccgatcacgg 2580
   gccacgtcaa atgcaggcca ctccgccaac accggcgcca ccaatggtcg tactacgggc 2640
   gcccagacca acatggagag caacgagtcc cccaggaacg tgccgataat ggtcaagcct 2700
   aagaccctct accagaaccc gcagaccccc acagttctcc cctcaactta ccacccgatt 2760
   aacaagtgga gctccgttaa gaatacctat ttaaaagagt tcctcgccga gtttatgggt 2820
   acgatggtca tgatcatctt cggcagcgcc gtcgtatgcc aggtcaacgt cgccggcaag 2880
   atccagcagg acaacttcaa cgtagccctc gacaacctca atgtcacggg ctcttccgcc 2940
   gagacaatcg acgccatgaa gagcctgact agcctagtct cctctgtcgc cggtggcacc 3000
   ttcgatgacg ttgccctcgg gtgggccgct gccgtggtaa tgggctactt ttgtgcaggt 3060
   ggctcggcaa tctctggagc ccacctgaac ccatcaatca ccctcgctaa cctcgtttat 3120
   cgaggctttc ccctgaagaa ggtcccatat tacttcgcag gccagctgat tggtgctttc 3180
   acgggagccc tcattctgtt catttggtac aagagggtcc ttcaggaggc atactcggac 3240
   tggtggatga acgaatccgt cgctggcatg ttttgtgtct tcccaaagcc ctacctgagc 3300
   tctgggcggc agttttttag cgagttcctc tgcggcgcca tgctccaggc cgggacattc 3360
   gccctgacgg atccctacac gtgcttgagc tcagatgtat ttcccctgat gatgttcatt 3420
   ctcattttca tcattaacgc ttcgatggca tatcagacag gtactgcgat gaacctggca 3480
   cgggatctgg gcccccgcct cgccctctac gcagtgggct tcgaccacaa gatgctctgg 3540
   gtccaccacc accacttctt ttgggtgccg atggtagggc cgtttattgg tgccctgatg 3600
   gggggcctcg tctacgacgt ctgtatctac caaggccacg aatcaccggt taactggtct 3660
   ctccccgtgt acaaggaaat gattatgcgc gcatggttta ggcgccccgg atggaagaag 3720
   cggaaccgag ctaggaggac ttctgacctc tccgacttct cttacaataa tgacgacgac 3780
   gaggaattcg gcgaacgcat ggcgcttcag aagacgaaga cgaagtctag catttccgac 3840
   aacgagaacg aggccggcga gaagaaggtt cagttcaagt ccgttcagcg agggaagcgg 3900
   accttcggcg gcatcccgac aattcttgag gaggaggata gcatcgagac tgccagcctc 3960
   ggcgccacga caacggattc tattggtctc tctgacacta gctctgagga cagccactac 4020
   ggcaacgcga agaaagtcac ctgaatgcat ggctttcgtg accgggcttc aaacaatgat 4080
   gtgcgatggt gtggttcccg gttggcggag tctttgtcta ctttggttgt ctgtcgcagg 4140
   tcggtagacc gcaaatgagc aactgatgga ttgttgccag cgatactata attcacatgg 4200
   atggtctttg cgatcagtag ctagtgagag agagagaaca tctatccaca atgtcgagtg 4260
   tctattagac atactccgag aataaagtca actgtgtctg tgatctaaag atcgattcgg 4320
   cagtcgagta gcgtataaca actccgagta ccagcaaaag cacgtcgtga caggagcagg 4380
   ctttgccaac tgcgcaacct tgcttgaatg aggatacacg gggtgcaaca tggctgtact 4440
   gatccatcgc aaccaaaatt tctgtttata gatcaagctg gtagattcca attactccac 4500
   ctcttgcgct tctccatgac atgtaagtgc acgtaggaaa ccatacccaa attgcctaca 4560
   gctgcggagc atgagcctat ggcgatcagt ctggtcatgt taaccagcct gtgctctgac 4620
   gttaatgcag aatagaaagc cgcggttgca atgcaaatga tgatgccttt gcagaaatgg 4680
   cttgctcgct gactgatacc agtaacacct ttgcttggcc gtctagcgct gttgattgta 4740
   ttcatcacaa cctcgtctcc ctcctttggg ttgagctctt tggatggctt tccaaacgtt 4800
   aatagcgcgt ttttctccac aaagtattcg tatggacgcg cttttggctg tattgcgtga 4860
   gctaccagca gcccaattgg cgaagtcttg agccgcactc gcatagaata attgattgcg 4920
   catttgatgc gatttttgag cggctgtttc aggcgacatt tcgcccgccc ttatttgctc 4980
   cattatatca tcgacggcat gtccaatagc ccggtgatag tcttgtcgaa tatggctgtc 5040
   gtgaataacc catcggcagc agatgataat gattccgcag cacgctagcg agctctccgg 5100
   agcccaaaat agaagcaccc gaattcaggg cctttttctc cacaccaatc accaaattca 5160
   ctgatttcaa ggttgttagc cgcctcttat cactaggtag actagcacct aattttaccg 5220
   atatgtgtag tccatacatg tatgagatat acatcatgat ctcgaagtaa aacccggtac 5280
   gaagaggtat aaaagccgcg gaacaacaac ttgcagatgc catcacgcaa gacgcagaca 5340
   cggctctctt ctcgcgggca tcaccagagc ccaatcacct ggtcctcagg tggccacttg 5400
   agcgccgtca gaatggccgc acagcagcag cagcatcacc acagatcttg gcctcaactc 5460
   ttgatatttc ggcgagaata caattactag tctagtctat ggaaattgga tgttgtgcgg 5520
   ctctttggag actggtagta cataagccac gaacatgata ttggcgggga aggtttagct 5580
   ggaaattgga acaagtcaga gaagtcgccg cgtattagct tggtgttagt agacacagca 5640
   agtcaagagt gggagttaga tttctgaatt gagtgacgca gatggatgta gtattcatta 5700
   ttgaagacag agcctttcca tcaacgtgca gagccatgaa atgcaatgac cttaaggagg 5760
   ggcatctctc cgctttccat ctgactgaga tactaacccg attccatctc cattttctag 5820
   cacacacata cctggagtca tacctcaacc ggccttcagt agctaaacag ctatgtagcg 5880
   catccgcgct aagccgctta ggcgataacg ttggccggtg tcggccatac aattcttgcc 5940
   tatgactctg tctcgactaa ctgattgacc aacgtcaatt ccttatcaaa gttttcgacc 6000
   ttgataactc tcgatcttaa tctcgagctt tttatttcct caacatcatt cacagtaacg 6060
   actcaagccc ttggacttgg ccagcaattg acacaaccac gcgccatctg ctccattggc 6120
   ctcatcgatc ccatcgactt cgtcagcctc gacaacgcat ccaacgcaat cacaaatact 6180
   tcaacatggc tgagaaaaag gcggacgccc cggcagccgg catgctctgg ggtggcaggt 6240
   tcacaggttg cagcctcgca aatctagctg cggattcagt atactgacct ctcgcgatgc 6300
   gcaggtgcaa ttgatcctct catgcacaag tacaatgcct ccatcgggta cgacaaggcc 6360
   ctgtacaagg aagatatcct tggatccatt gcctttgcga gggccaattc aaaggctggc 6420
   atcatcagcg aagacgaatt ccaggcgatc gagcgtggct tgctccaagt gatggaagaa 6480
   tggaagcagg gcactttcgc catcatgccg aatgacgaag atgtgagagg gcacactcga 6540
   ttctaatctc aaacttcttt cgaggctgac acaatggacc agattcatac ggcaaacgaa 6600
   cgtcgcctgg gagaggtcat cggcaaggac atcgcgggaa agcttcacac aggtcgcagc 6660
   cgcaacgagc aagttgtctg tgatatgcgc atgtggcttc gcgatcggat ccgcgaaatc 6720
   gacagccaac tcgttgcttt cctcaaggtc attatcacac gtgccgaatc tgaaatgtaa 6780
   gcccagccgt acctttccag ctaaatgccc cccccccccc cccccccctc tcttactgac 6840
   ggacaacctc agcgactaca tcatgcccgg atacacccat ctccagcgcg ctcagccagt 6900
   tcggtggtct caatggctca tgtcctatgc cgctgcgttc aagcaggatc tcgagcggtt 6960
   gcgtcaagtc ttcgagaggg tcaacctcag tccacttggc tgtggcgcct tggccggcaa 7020
   tccattcgga atcgaccgag actcaatagc tgcggagctt ggcttcagcg gaatcacctt 7080
   gaactccatg aatacttcgg ccgaccgcga tttcctcgtc gatttcctgt cctggaactc 7140
   catcttcacc aaccacgtca gcaggtgggc tgaggacctc atcatctact cgacctctga 7200
   gttcagtttc gtccgtctgg ccgacgccta ttccacgggc agctctctca tgcccaacaa 7260
   attcaacccg gactcgttgg agcttcttcg cggcaagtca gggcgtgctt tcggccaaat 7320
   ggctggtttg atgatggtga gtcctcatat caaacatctc agccagtttg acttggcttt 7380
   tgagggtgac cgcggccgcc gttccggtaa ttaactggcc tcatgggcct tcacttcact 7440
   gcccgctttc cagtcgggaa acctgtcgtg ccagctgcat taacatggtc atagctgttt 7500
   ccttgcgtat tgggcgctct ccgcttcctc gctcactgac tcgctgcgct cggtcgttcg 7560
   ggtaaagcct ggggtgccta atgagcaaaa ggccagcaaa aggccaggaa ccgtaaaaag 7620
   gccgcgttgc tggcgttttt ccataggctc cgcccccctg acgagcatca caaaaatcga 7680
   cgctcaagtc agaggtggcg aaacccgaca ggactataaa gataccaggc gtttccccct 7740
   ggaagctccc tcgtgcgctc tcctgttccg accctgccgc ttaccggata cctgtccgcc 7800
   tttctccctt cgggaagcgt ggcgctttct catagctcac gctgtaggta tctcagttcg 7860
   gtgtaggtcg ttcgctccaa gctgggctgt gtgcacgaac cccccgttca gcccgaccgc 7920
   tgcgccttat ccggtaacta tcgtcttgag tccaacccgg taagacacga cttatcgcca 7980
   ctggcagcag ccactggtaa caggattagc agagcgaggt atgtaggcgg tgctacagag 8040
   ttcttgaagt ggtggcctaa ctacggctac actagaagaa cagtatttgg tatctgcgct 8100
   ctgctgaagc cagttacctt cggaaaaaga gttggtagct cttgatccgg caaacaaacc 8160
   accgctggta gcggtggttt ttttgtttgc aagcagcaga ttacgcgcag aaaaaaagga 8220
   tctcaagaag atcctttgat cttttctacg gggtctgacg ctcagtggaa cgaaaactca 8280
   cgttaaggga ttttggtcat gagattatca aaaaggatct tcacctagat ccttttaaat 8340
   taaaaatgaa gttttaaatc aatctaaagt atatatgagt aaacttggtc tgacagttat 8400
   tatttaccca ccactttggt aatttcgcct ttcacgtaat gcacaaattc ttccagctga 8460
   tccgcacggc tggccagacg atcttcttcc tggcccagat acgcctgacg cgcttccaga 8520
   atcaccggct gatactgcgc cggcagacgt tccatggccc aatccgccgc cacatctttc 8580
   ggcgcaattt tgccggtcac cgcgctatac caaatacggc tcagggtcag caccacgttg 8640
   cgttcatcac ccgcccaatc cggcgggctg ttccacaggg tcagggtttc gttcagcgct 8700
   tcaaacaggt cctgttccgg caccggatca aacagttctt ccgctgccgg accaaccagc 8760
   gccacgctat gttcacgcgc tttggtcagc agaatggcca gatcgatatc aatggtcgcc 8820
   ggttcaaaaa tgccggccag aatatcgtta cgctgccatt cgccaaactg cagctcacgt 8880
   ttcgccggat aacgccacgg aataatgtca tcatgcacca cgatggtcac ttccaccgca 8940
   cgcagaattt cgctttcacc cgggctcgcg ctggtttcca gcagatcgtt aatcagcgca 9000
   cgacgggtgg tttcatccag acgcacggtc acggtcacca gcagatcaat atcgctatgc 9060
   ggtttcaggc cgccatccac cgcgctgcca tacagatgca cggccagcag ggtcggttcc 9120
   agatgacgtt caatcacgcc caccacttca gacagctggg tgctcacttc cgcaatcacc 9180
   gcttcacgca taatgttcag tttggttttc ggacggctgc cctgctgggt cacatcgttg 9240
   ctgctccaca gcatcaggca acggcccacc gcataacgcg cctgctgttt gctcgcacgc 9300
   ggcatgctct gcacgccaaa aaagcaatca tagcacgcca taaacaccgc caccgcacca 9360
   ttaccgccgc tacgttcggt cagggtacgg ctccaattac ggctacgcat actcttcctt 9420
   tttcaatatt attgaagcat ttatcagggt tattgtctca tgagcggata catatttgaa 9480
   tgtatttaga aaaataaaca aataggggtt ccgcgcacat ttccccgaaa agtgccac 9538
<210> 10
   <211> 11413
   <212> DNA
   <213> artificial sequence
<220>
   <223> pKR_Rpyr4err1
<400> 10
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtggccgct acagggcgct cccattcgcc attcaggctg cgcaactgtt     180
   gggaagggcg tttcggtgcg ggcctcttcg ctattacgcc agctggcgaa agggggatgt     240
   gctgcaaggc gattaagttg ggtaacgcca gggttttccc agtcacgacg ttgtaaaacg     300
   acggccagtg agcgcgacgt aatacgactc actatagggc gaattgagtg aaggccgtca     360
   aggcctaggc gcgccagcgg ccgccagatc tcgagatagt atctcattca gtaaaatact     420
   cgtacgagtt tgagggggcg cgaggctaag ctgcaagcgg gcgcggcaac actacctgta     480
   tgtattaata aagcagcaga gagagagaga gccctggact gccggcaatc ctgagaaggg 540
   gggatcgacc ggaccatctg cacgcagcag ggctgctgct gccaacatgg agagactaaa 600
   gatgggattg gtggtacata gcaagacgct tggatgcttg ttgacgggct gctccaataa 660
   gcaagctcac aagcagtgct ttgggggtcc gcacggacgg tcgagagggc tcggacggct 720
   gatattgcag acctggacga gtcagttgtg caggtaggca ctcgtatgtg tgtctttgtg 780
   cttgatcaac cttgagtaag tattattagg gtgatggctt aggtaggtag gttaggtagg 840
   tagtttaggt acagggaggt gaggagggga gacgtccagc tgccatatgc gccacttgca 900
   gcctcaactc tgcaaaagcc agccccgatc acggcatgtc gtacatactg taaggtagta 960
   cctagtagta gaacgaatgc taaaggtgca gagcaactaa cttgagcaaa accccccctc 1020
   cccccatcga gattgattgc cctctacgct gaaaatagtc tcgccagccg gcgacgctta 1080
   gccgtggcgc taccgcgaca tggcgcttgt tagcctgcgg tccaggttga tgcgaagcat 1140
   gtcgcagggc gggtctcgtg cgtgccgaca gccagcagag agacagaaga gagcgagaga 1200
   gcgagagagc gagagagaga gagagagaga aagacgcctc gggggtttgg ctggtggctt 1260
   cgatgcttgt ccgtgtcaag ggttcacgaa agacgggacg gctgagttgg tgactggcgg 1320
   cggtggcatc gaggctggag tccaggcaca tgcatggcgc aaggcatgtc aggccatgtc 1380
   gtgtcatgcc ccggcacagc tgaagggaat actactattg agagctgttc tccaaggcgt 1440
   caagcatgtg agcaaaaact cgagttgtct agactcgact cgacttgcgg ctgcagtaca 1500
   acaatacagt actactcgta cacgtatgta cagcaaggag caggtggatc agcaaagttg 1560
   catgataatg gactggaccg gcaacgctca aagttcgcct tgcagctccg gcatgaaggg 1620
   cagacccccg ccattattag cgatgactag ccgtggaggc cactttggct ttagtgattt 1680
   aaaccccgag attcatgctc gatgctctac aggtatgtat gtactagctc agctcagcct 1740
   tagtaggtag gtattgaagt ctggtggtgc gaggaggacc tgcaggtggg agttttgggg 1800
   gaaaaagatg atgctcacca actaaaataa atagtcaggc cacacacaca acctactgag 1860
   cagaaccacc acaaccagtg aagagctact ccggaattct cgactccgga gagttgggca 1920
   aaatcaggcg cggaccagcg ccaaggtgtg ggtggattgg gtcgtaagaa aaagaggcat 1980
   ccctacatgc agcaaccggc tggagacgag ccagcccgag cactggcagc ctcagaccag 2040
   aggcatctcg accacggctt caagtatcag actgggagct gtgccgagtt tgctggctac 2100
   ttacctagtc aaaaaggtcg ccgctgactg tctgtgtcat attaacgagc caggtctcaa 2160
   ccttctgtcg aatgaaatgg aaagcggcga atgactcttc ggggttggcc agaaacaacg 2220
   acgaccactg agggatcggg acgatgtaca tctactgcga tatgccgggc tcgaatgtga 2280
   ctgcacacac cacacacaca cacacacacg gacacatctg agcagtacta tcaaagcggt 2340
   gggagtgcat ctgcactggg cacccttact gtaggtagga aggataccta aggtagaggg 2400
   ctcgctgctg caagttgata tgacggccac caatcggaag ggtgtcgtat ctcacatcac 2460
   cagcgtcagg acacgcttca caaaattccc acaagttcca gttgaaatga cccgtaggag 2520
   agcgcctcga gggacagaat gtacagtact atactaccta ccttagatgc catcacccct 2580
   tcgcaggcca tggaatttct ggcaagtcgc acccttcagc catgcctgtc cgccatcatc 2640
   ataccagatc gtgtcgtggc gggcacttgg gaaggacgag ctccaaccgc cgccgctgat 2700
   tggcctgatc cgccacagcc actggcgctc gcggcgagga aaaaaaaaaa tcttgcgggt 2760
   gtgcagcagc attgagtgga aagcgggagc cgctgcacga cgctgattcg tccgggctgc 2820
   ctcgttaggg ctagaccagc aggcgggcgc cgcttatttt gcccctcctc aggctttttt 2880
   tcacaagccc cgttttgctg ccccgcgcgt ggcagaaaaa aaaaatacag cacgagccac 2940
   tggcacttgc acccctccac ccctccgccg accacctttt aaatctcctc ctcccaccat 3000
   ctgctttcga aattttctct cttctgctct tcgtctcgca tacccggttc aagcatccga 3060
   tctgcgaatt tgtatgtgct gcctctccct ctgaccttct ggtctggtga taccatcctc 3120
   cctcagtttg gatcatcgcc ttattcttct tccctcttct gcatctgctt cctgctcgtt 3180
   tgaggaacat cgccagctga ctctgcttgc ctcgcagcga tctagtcaag aacaacacag 3240
   ctctcacgct acatcacact taagtctaga atgtcttccg gaaggaccgt caccctcaac 3300
   accggctaca agatccccca gatcggctac ggcacctggc aggccgctcc cggcgaggtc 3360
   ggcgctggtg tctttgaggc cctcaaggtt ggctaccgcc acctcgacct ggccaaggtc 3420
   tacggcaacc agaaggaggt tggtgagggc atcaagaagg ctcttgctga ggtccccggc 3480
   ctgaagcgcg aggatatttt catcacctcc aagctgtgga acaactccca caagcccgag 3540
   gacgtcgagc ccgctctcga cgacaccctg gccgagcttg gcctcgacta ccttgacctc 3600
   tacctcatcc actggcccgt tgcctttgct cccggcgccg acctcttccc caagtccgag 3660
   gacggctccg aggtgcagct caaccagaat gtgtccattg tccagacctg gaaggccatg 3720
   accgagctgc ccaagtccaa ggtccgctcc gtcggtgtct ccaactttac catagagcac 3780
   ctcgacgccg tcatcgaggc caccggcgtc gtccccgccg tcaaccagat cgagcgccac 3840
   ccccgcctcc ccaaccagcc tctgatcgac tactgcgcca agaagggcat catcatcacc 3900
   gcctactccg cctttggcaa caacacaaag ggcctgcccc tgctcgtcag ctccgacgag 3960
   gtcaaggccg tcgccgacaa cctgtccaag aagcagggca agaccgtcac tcccgcccag 4020
   gtcatcctcg cctggtccca gattggtggc cacaccgtca ttcccaagtc cgtcaccaag 4080
   gcgcgcattg cggagaactt ccaggaggtt gagctggatg acgaggccat tgctgcgctg 4140
   aacaagttgg gcgagaagcc tcagcggttc aacattcctt acacctacaa gcctaggtgg 4200
   aacattaacc tgttcaacac cgaggaggag aaggccgctg cccacactgc tgtcatcaag 4260
   ctgtaaacta gtgaattctg gatcctttcg tgaccgggct tcaaacaatg atgtgcgatg 4320
   gtgtggttcc cggttggcgg agtctttgtc tactttggtt gtctgtcgca ggtcggtaga 4380
   ccgcaaatga gcaactgatg gattgttgcc agcgatacta taattcacat ggatggtctt 4440
   tgcgatcagt agctagtgag agagagagaa catctatcca caatgtcgag tgtctattag 4500
   acatactccg agaataaagt caactgtgtc tgtgatctaa agatcgattc ggcagtcgag 4560
   tagcgtataa caactccgag taccagcaaa agcacgtcgt gacaggagca ggctttgcca 4620
   actgcgcaac cttgcttgaa tgaggataca cggggtgcaa catggctgta ctgatccatc 4680
   gcaaccaaaa tttctgttta tagatcaagc tggtagattc caattactcc acctcttgcg 4740
   cttctccatg acatgtaagt gcacgtagga aaccataccc aaattgccta cagctgcgga 4800
   gcatgagcct atggcgatca gtctggtcat gttaaccagc ctgtgctctg acgttaatgc 4860
   agaatagaaa gccgcggttg caatgcaaat gatgatgcct ttgcagaaat ggcttgctcg 4920
   ctgactgata ccagtaacac ctttgcttgg ccgtctagcg ctgttgattg tattcatcac 4980
   aacctcgtct ccctcctttg ggttgagctc tttggatggc tttccaaacg ttaatagcgc 5040
   tagtagtaga aatggagggt aaactgaaca tcagtcaaaa gacgtcgaga cgcagatact 5100
   actagatacc tatcccagcg acggaaaatg gacaaggtgt cgccgccgcc aagctctcag 5160
   atgcgctatc gtgcatgatg cctctcatca acaaacggcg ccagtgacgg gctagccgtg 5220
   ggattgactc gagctcgcct cttctttgtg cttttctcct gtcgacatcc cggcttgcgc 5280
   ttggacctcg ccgggacgtg caaacgaaca catcactttc aaagagagtg tttgatgctc 5340
   acgctcggat gctccagccc ccccttgctt gcttgtccat cattgaggct cgaaggggca 5400
   gttgctcccc attccctctg ctccaacagg aactgtaacc gaaaaaaaag ccatcctcct 5460
   tcttcctctt cctcttccgc ctcctcctct tcctcctcct actaatacct actcctcctc 5520
   tcctcatcga ctagactgac ccccccggtt gggcccctcg tcccgtctcc aacagagcac 5580
   caccagacaa agacccctgc ccgcgcgaat ccagaccccc ccagcaattc cgggcctcgt 5640
   tgatcctcct ctactgtagt tgtacataca tacctaccga ctgcattgca ttggtacagc 5700
   tgcaggcact tccaggcacg gccaccaaat tgcagcggcc cttgcttgct tgcttggttc 5760
   gagaactagg atctgtgtct tttgccttgc cttgtcttgt ctgggttcct gctcgtctgc 5820
   ggcaatcgga acgccgccag tgcggtgcca agcaaaccag ccaaggtagg tacctaccac 5880
   taggcttctt ttctcgttgt ctcactctct cttttcctct ttgtcctctc ttatccccat 5940
   cttttctctc tctctctgct cctttcctaa ccacttccct acctttctct ttttcctttt 6000
   cttgtcatct ccatcttggc tgacgaaaaa ggtctgactg ggtaggtatt atctggcaga 6060
   cttgtgtgta tcattcaccc tatttctgct tcatagtaca tgtactgtac ctgaacggct 6120
   caaccgctat ttacgactct tatttttttg tggcgttggt cacgtttgcc agctgttgtc 6180
   cgtctttcta gggctcctca aacttgacct gaccgagctc cctttctgga cccggtgggc 6240
   ttcacttcca gctgctgagc gacctgagcc gaacatcctc agtccttgtc cagcgcaatt 6300
   cattttcttt ccttttcttt ttttttattc ctttctttac ttttattctc tctttttctc 6360
   ctcttcctct tcttcttctt tctcctcctc ctccatatcc tcactctcgt ctccctcatt 6420
   actaccctct cggctcctca ggtccaccaa ccctcccgca cccaaacctc tgccgctgaa 6480
   acccattcgg tggtcgccgt tttttttttt ttttttttct cacccccaaa gtcgcaatat 6540
   cgggtatcgc cgccggcatt gaatcgcctt ctccgctagc atcgactact gctgctctgc 6600
   tctcgttgcc agcgctgctc cctagaattt tgaccagggg acgagcccga cattaaagca 6660
   actccctcgc ctcgagacga ctcggatcgc acgaaattct cccaatcgcc gacagttcct 6720
   actcctcttc ctcccgcacg gctgtcgcgc ttccaacgtc attcgcacag cagaattgtg 6780
   ccatctctct cttttttttc cccccctcta aaccgccaca acggcaccct aagggttaaa 6840
   ctatccaacc agccgcagcc tcagcctctc tcagcctcat cagccatggc accacacccg 6900
   acgctcaagg ccaccttcgc ggccaggagc gagacggcga cgcacccgct gacggcttac 6960
   ctgttcaagc tcatggacct caaggcgtcc aacctgtgcc tgagcgccga cgtgccgaca 7020
   gcgcgcgagc tgctgtacct ggccgacaag attggcccgt cgattgtcgt gctcaagacg 7080
   cactacgaca tggtctcggg ctgggacttc cacccggaga cgggcacggg agcccagctg 7140
   gcgtcgctgg cgcgcaagca cggcttcctc atcttcgagg accgcaagtt tggcgacatt 7200
   ggccacaccg tcgagctgca gtacacgggc gggtcggcgc gcatcatcga ctgggcgcac 7260
   attgtcaacg tcaacatggt gcccggcaag gcgtcggtgg cctcgctggc ccagggcgcc 7320
   aagcgctggc tcgagcgcta cccctgcgag gtcaagacgt ccgtcaccgt cggcacgccc 7380
   accatggact cgtttgacga cgacgccgac tccagggacg ccgagcccgc cggcgccgtc 7440
   aacggcatgg gctccattgg cgtcctggac aagcccatct actcgaaccg gtccggcgac 7500
   ggccgcaagg gcagcatcgt ctccatcacc accgtcaccc agcagtacga gtccgtctcc 7560
   tcgccccggt taacaaaggc catcgccgag ggcgacgagt cgctcttccc gggcatcgag 7620
   gaggcgccgc tgagccgcgg cctcctgatc ctcgcccaaa tgtccagcca gggcaacttc 7680
   atgaacaagg agtacacgca ggcctgcgtc gaggccgccc gggagcacaa ggactttgtc 7740
   atgggcttca tctcgcagga gacgctcaac accgagcccg acgatgcctt tatccacatg 7800
   acgcccggct gccagctgcc ccccgaagac gaggaccagc agaccaacgg atcggtcggt 7860
   ggagacggcc agggccagca gtacaacacg ccgcacaagc tgattggcat cgccggcagc 7920
   gacattgcca ttgtgggccg gggcatcctc aaggcctcag accccgtaga ggaggcagag 7980
   cggtaccgat cagcagcgtg gaaagcctac accgagaggc tgctgcgata ggggagggaa 8040
   gggaagaaag aagtaaagaa aggcatttag caagaagggg gaaaagggag ggaggacaaa 8100
   cggagctgag aaagagctct tgtccaaagc ccggcatcat agaatgcagc tgtatttagg 8160
   cgacctcttt ttccatcttg tcgatttttg ttatgacgta ccagttggga tgatggatga 8220
   ttgtacccca gctgcgattg atgtgtatct ttgcatgcaa caacacgcga tggcggaggc 8280
   gaactgcaca ttggaaggtt catatatggt cctgacatat ctggtggatc tggaagcatg 8340
   gaattgtatt tttgatttgg catttgcttt tgcgcgtgga gggaacatat caccctcggg 8400
   catttttcat ttggtaggat ggtttggatg cagttgtcga cgatatcagc ttccatatat 8460
   acggaattgt tcaattgagt attacctgat ggctatttgt gtcgatcctt tgtgacattg 8520
   acatgtgatg gcgcctgtac ctctaaatgt atgcagcgta acatcttcag agcacggcgg 8580
   catgttctcc cctctacaat attcagataa gcagtacttg tgctgtgaga aacccattga 8640
   tcgctggtgg caaccaaggc gatatgtctc caggagagga aattgcacga tcattgatcg 8700
   tttgtgctac tcgtttcgtg tccaagcgaa cagagtatta atatatacca ttatatcata 8760
   tgcatcgtgg agtcgttact atgcagccta acctaaggta cctacaaatc ggcatcccgg 8820
   agttaacgcg gaggctctca tcgccgggcg tctccaccaa ttcgacatat caatgctaat 8880
   cgggcgcaaa ggctccaacg cggatgctgg tcgcattgag gttccgcatt ccttttcctc 8940
   cttccgaccc tctttcttac atctcactct cacatcccgt ttgccggccc gctccccacc 9000
   gagacacgaa acggacggca acaatcaacc gtcccccggg aacagcgacc tgacctcccc 9060
   tccccccata tacgattacg agctgaacga gacgagagag agagagagag agaagaagaa 9120
   gacagagaag aggcatggcc gacattgagg tggcagtcaa cgtacgggcc gagcggggcc 9180
   cgactaagaa gcggagacgg atcgtcatct cgtgcaccga gtgccatcga aggaagcaaa 9240
   aggtcaggct tctttcctac caagaaacaa agtgaagctt attaattaac tggcctcatg 9300
   ggccttcact tcactgcccg ctttccagtc gggaaacctg tcgtgccagc tgcattaaca 9360
   tggtcatagc tgtttccttg cgtattgggc gctctccgct tcctcgctca ctgactcgct 9420
   gcgctcggtc gttcgggtaa agcctggggt gcctaatgag caaaaggcca gcaaaaggcc 9480
   aggaaccgta aaaaggccgc gttgctggcg tttttccata ggctccgccc ccctgacgag 9540
   catcacaaaa atcgacgctc aagtcagagg tggcgaaacc cgacaggact ataaagatac 9600
   caggcgtttc cccctggaag ctccctcgtg cgctctcctg ttccgaccct gccgcttacc 9660
   ggatacctgt ccgcctttct cccttcggga agcgtggcgc tttctcatag ctcacgctgt 9720
   aggtatctca gttcggtgta ggtcgttcgc tccaagctgg gctgtgtgca cgaacccccc 9780
   gttcagcccg accgctgcgc cttatccggt aactatcgtc ttgagtccaa cccggtaaga 9840
   cacgacttat cgccactggc agcagccact ggtaacagga ttagcagagc gaggtatgta 9900
   ggcggtgcta cagagttctt gaagtggtgg cctaactacg gctacactag aagaacagta 9960
   tttggtatct gcgctctgct gaagccagtt accttcggaa aaagagttgg tagctcttga 10020
   tccggcaaac aaaccaccgc tggtagcggt ggtttttttg tttgcaagca gcagattacg 10080
   cgcagaaaaa aaggatctca agaagatcct ttgatctttt ctacggggtc tgacgctcag 10140
   tggaacgaaa actcacgtta agggattttg gtcatgagat tatcaaaaag gatcttcacc 10200
   tagatccttt taaattaaaa atgaagtttt aaatcaatct aaagtatata tgagtaaact 10260
   tggtctgaca gttattattt acccaccact ttggtaattt cgcctttcac gtaatgcaca 10320
   aattcttcca gctgatccgc acggctggcc agacgatctt cttcctggcc cagatacgcc 10380
   tgacgcgctt ccagaatcac cggctgatac tgcgccggca gacgttccat ggcccaatcc 10440
   gccgccacat ctttcggcgc aattttgccg gtcaccgcgc tataccaaat acggctcagg 10500
   gtcagcacca cgttgcgttc atcacccgcc caatccggcg ggctgttcca cagggtcagg 10560
   gtttcgttca gcgcttcaaa caggtcctgt tccggcaccg gatcaaacag ttcttccgct 10620
   gccggaccaa ccagcgccac gctatgttca cgcgctttgg tcagcagaat ggccagatcg 10680
   atatcaatgg tcgccggttc aaaaatgccg gccagaatat cgttacgctg ccattcgcca 10740
   aactgcagct cacgtttcgc cggataacgc cacggaataa tgtcatcatg caccacgatg 10800
   gtcacttcca ccgcacgcag aatttcgctt tcacccgggc tcgcgctggt ttccagcaga 10860
   tcgttaatca gcgcacgacg ggtggtttca tccagacgca cggtcacggt caccagcaga 10920
   tcaatatcgc tatgcggttt caggccgcca tccaccgcgc tgccatacag atgcacggcc 10980
   agcagggtcg gttccagatg acgttcaatc acgcccacca cttcagacag ctgggtgctc 11040
   acttccgcaa tcaccgcttc acgcataatg ttcagtttgg ttttcggacg gctgccctgc 11100
   tgggtcacat cgttgctgct ccacagcatc aggcaacggc ccaccgcata acgcgcctgc 11160
   tgtttgctcg cacgcggcat gctctgcacg ccaaaaaagc aatcatagca cgccataaac 11220
   accgccaccg caccattacc gccgctacgt tcggtcaggg tacggctcca attacggcta 11280
   cgcatactct tcctttttca atattattga agcatttatc agggttattg tctcatgagc 11340
   ggatacatat ttgaatgtat ttagaaaaat aaacaaatag gggttccgcg cacatttccc 11400
   cgaaaagtgc cac 11413
<210> 11
   <211> 4768
   <212> DNA
   <213> artificial sequence
<220>
   <223> mpdh deletion cassette
<400> 11
   aaagacatga accaggctgg agacgagata gtcaggcacc agatggcgag atgggacgag 60
   gaagaaccat ggacgaggcg aagaatgagg caaagttgga atgacggact gcagtgagtg    120
   gatggagttc aaagtacctg ggtacctaca tactgcgtgc atgggccacg ataccggcaa    180
   ggaccagcga cacggcaggt acgaagcacg aaccagcaag cacgaaccag catcgtccgt    240
   aaatgcaggt gcgttcctgc cttctagacg gcggtaccag ataagagacg ctgcaccgca    300
   cataacggaa ggtcactcga cccgctttca aggtggctct tgcgccacgg cctgctggaa    360
   gccacgcccc atgattggcc agcagagagg ctcatgccac tggacagctg tactggtacc    420
   tgctgcttct cacctcatgc cctgatgcgt tgttggactg atgggatggt ctcacccgta    480
   cgacgtagca cgaggcctgg accactctgg cattgacacg gcactcaatg caacaatggc    540
   atccgatggc cgctgtatgt tgtatcatgt agagcatcga gtacttccac tcgtacctgt    600
   acctgcgctc tcgaatgggg accccccctg tggatctggc ggtacaagct gcatgtactc    660
   cctggactcg gtccacaccc cccctgctgt gacgatggtc gatcagcttc aaagtccctc    720
   catcttcttc gcctgttgga cccgattgag gcgccatcgt catcgggacc ccctacctga    780
   cctctggcct gtaatactgt cctggctgta tgtcctgcta ccacctggag cagcctttcg    840
   gtggctctcg tgcttctcgt ggactctgtc aaactcatcc tcgaattatt ataaagctgg    900
   ccggactccc gctcctccta cttgactctt agttcctctc catcatcaac cagctttcac    960
   cacacaaaaa ctcccatttc tctttctctt tctcttcctc ctacaaccac cacatcgcca   1020
   actcttcctt ctgcaactcc aactccttca cttctcttct ctgttctact cttctccacc   1080
   tacttcacct accgcaatcg agagctacct tacatcaata tggccagcac ctcttcggcg   1140
   atacatactc gccaccccag ccggggcgat tgtgtgtact aggtaggctc gtactatacc   1200
   agcaggagag gtgctgcttg gcaatcgtgc tcagctgtta ggttgtactt gtatggtact   1260
   tgtaaggtgg tcatgcagtt gctaaggtac ctagggaggg attcaacgag ccctgcttcc   1320
   aatgtccatc tggataggat ggcggctggc ggggccgaag ctgggaactc gccaacagtc   1380
   atatgtaata gctcaagttg atgataccgt tttgccagga ttaggatgcg agaagcagca   1440
   tgaatgtcgc tcatccgatg ccgcatcacc gttgtgtcag aaacgaccaa gctaagcaac   1500
   taaggtacct taccgtccac tatctcaggt aaccaggtac taccagctac cctacctgcc   1560
   gtgcctacct gctttagtat taatctttcc acctccctcc tcaatcttct tttccctcct   1620
   ctcctctttt ttttttcttc ctcctcttct tctccataac cattcctaac aacatcgaca   1680
   ttctctccta atcaccagcc tcgcaaatcc tcaggttagt attactacta ctacaatcat   1740
   caccacgatg ctccgcccga cgatgcggct tctgttcgcc tgcccctcct ctcactcgtg   1800
   cccttgacga gctaccccgc cagactctcc tgcgtcacca atttttttcc ctatttaccc   1860
   ctcctccctc tctccctctc gtttcttcct aacaaacaac caccaccaaa atctctttgg   1920
   aagctcacga ctcacgcaag ctcaattcgc agatacaaat ctagaatgaa aaagcctgaa 1980
   ctcaccgcga cgtctgtcga gaagtttctg atcgaaaagt tcgacagcgt ctccgacctg 2040
   atgcagctct cggagggcga agaatctcgt gctttcagct tcgatgtagg agggcgtgga 2100
   tatgtcctgc gggtaaatag ctgcgccgat ggtttctaca aagatcgtta tgtttatcgg 2160
   cactttgcat cggccgcgct cccgattccg gaagtgcttg acattgggga attcagcgag 2220
   agcctgacct attgcatctc ccgccgtgca cagggtgtca cgttgcaaga cctgcctgaa 2280
   accgaactgc ccgctgttct gcagccggtc gcggaggcca tggatgcgat cgctgcggcc 2340
   gatcttagcc agacgagcgg gttcggccca ttcggaccgc aaggaatcgg tcaatacact 2400
   acatggcgtg atttcatatg cgcgattgct gatccccatg tgtatcactg gcaaactgtg 2460
   atggacgaca ccgtcagtgc gtccgtcgcg caggctctcg atgagctgat gctttgggcc 2520
   gaggactgcc ccgaagtccg gcacctcgtg cacgcggatt tcggctccaa caatgtcctg 2580
   acggacaatg gccgcataac agcggtcatt gactggagcg aggcgatgtt cggggattcc 2640
   caatacgagg tcgccaacat cttcttctgg aggccgtggt tggcttgtat ggagcagcag 2700
   acgcgctact tcgagcggag gcatccggag cttgcaggat cgccgcggct ccgggcgtat 2760
   atgctccgca ttggtcttga ccaactctat cagagcttgg ttgacggcaa tttcgatgat 2820
   gcagcttggg cgcagggtcg atgcgacgca atcgtccgat ccggagccgg gactgtcggg 2880
   cgtacacaaa tcgcccgcag aagcgcggcc gtctggaccg atggctgtgt agaagtactc 2940
   gccgatagtg gaaaccgacg ccccagcact cgtccgaggg caaaggaata atgcatgtgc 3000
   tgtgttcctc agaatgggcc ccagaagggc gtcgagcatt gtctatgaat gcaaacaaaa 3060
   atagtaaata aatagtaatt ctggccatga cgaatagagc caatctgctc cacttgacta 3120
   tccttgtgac tgtatcgtat gtcgaaccct tgactgccca ttcaaacaat tgtaaaggaa 3180
   tatgagctac aagttatgtc tcacgtttgc gtgcgagccc gtttgtacgt tattttgaga 3240
   aagcgttgcc atcacatgct cacagtcact tggcttacga tcatgtttgc gatctttcgg 3300
   taagaataca cagagtaacg attatacatc catcgctttc tatgattagg tactcagaca 3360
   acacatggga aacaagataa ccatcgcatg caaggtcgat tccaatcatg atctggactg 3420
   gggtattcca tctaagccat agtacccttg cgatggctct tctggcgttc tagctcggca 3480
   tcactcacgg cgctgagtgg tgaccggcct actgcatctg gctttgggct tcttttgatc 3540
   ttttacttct actttttctc ttctacaact tgtgttaaga atgcacgaat gaacggcaag 3600
   gcgctgggtt ccgggttaat agagcatcgt ctcttttgtt ttctattttt tttgtactct 3660
   gctagggcat tccttgacgg gaacaaaagg tgctcgggat atcttcatgg gaagcttcag 3720
   tctatagatg gactgagcag gcagcataac gggtcaagat accacatcaa taaaagcaga 3780
   cttgatcatg accttccttt tcatccatca ttgcaatgtc ctcgtagatg tagctctagt 3840
   agcaggcaat ctaaatgtga ggtcaccatg atctcaagct gaagtttcca cagccagtag    3900
   tagacttctt aacgatgcaa atgcagccac cccgtccaat acggctcggt aggaacattt    3960
   cctccaatcc gtcaaccaac ccatctcacg ttccggctcg gcagctaatt aatcagccca    4020
   ggccatgaca ggcgacacat tggatggaag ttccgtcaca cacatccgct aaagcatgcc    4080
   ataccctttg ctagcacacc acccacaccc aaccagtcat gccgttccgt cccaccaccc    4140
   cctcttaatc ttgggtacgt atatggatgg atggatggac agacggctgg aaggacggat    4200
   ggatggatgc atccctgaga attacccaat taggaagggc acgatgtgtg gccggctgat    4260
   tggtccctga gcatttcatt cctagtgccc ttctttttcc ttcttcttct attttctcaa    4320
   ggggcagcct tacgctaggt attccaatac gtaacatgtt gtctcgaggt atgtcactgc    4380
   agagcaggtc aaagcaagac aaggctgagt ctacaggcac ctgcctccat tctcgtctct    4440
   ccgcatagta gtagtactac ctatttaacc aaatcccgga ctacgccgac catggacaac    4500
   atgatgtgag acacatctgg acagaagtgg gcaatgccat ttacagcctc atgcatgcca    4560
   cttcagcagc ttgagccctt gctgcagcat tatcacagca cctactggga cagtacatag    4620
   gtagtaggta ggaagtatgc aggtagcact acatggacat gccccctcgt ggccccctat    4680
   ttctgcgcgc atttcacgca cgacaattcc tcacacgagt tggggaacgg aaagattact    4740
   gacaggccct tgttttatgc cgactcgg                                        4768
<210> 12
   <211> 1615
   <212> DNA
   <213> Trichoderma reesei
<400> 12
   ctacaaccac cacatcgcca actcttcctt ctgcaactcc aactccttca cttctcttct        60
   ctgttctact cttctccacc tacttcacct accgcaatca tggccaacgc aactggcaag       120
   aaggcagtcc actttggcgc cggcaacatc ggccgtggct tcgttgcttg cttcctccac       180
   aactcgggct atgaggtcgt ttttgccgac gtcaacggag acctgattga taccatcaac       240
   tctaccccct cctacaaagt catcgaggtc ggctccgagg gcacctcaga aaacgtcatc        300
   accaactacc gggccatcaa ctctcggact cacgaggaag acctcgttga ggagatccgc       360
   actgccgacg tcgtcacctg ctccgtcggc cccaacatcc tcaagttcat cgctcccgtc       420
   atcgcaaagg gcatcgaccg ccgctccgac gacgagacgc ccctgcacgt cattgcctgc       480
   gagaacgcca ttggcgccac cgacacgctg gccagctaca tcaaggatgc caagaacacg       540
   gatcctgccc gtctcgagac gcaccacctg cgtgcccgct acgccaactc ggctattgac       600
   cggatcgtgc cggcgcagga ccccaatgcc ggccttgacg tgaagctgga gaagttcttt       660
   gagtgggttg tcgacaagac cccctttgag gacattggca tcccgcccat tgagggcatc       720
   cagtgggtcg acaacctgga gcccttcatc gagcgcaagc tgtacacggt caacactggc       780
   cacgctacgg ctgcctacca cggctactac cgcagaaagc gcaccgtcta cgatgctctc       840
   caggacaagg agatcatggc tgaggtccgt ggcgccctga ccgagaccaa gaacctgctc       900
   gttgccaagc acggcatcga cgaggaggcc cagctcgcct acatgaacaa gattatcaag       960
   cgcatcggca acccccattt ggaggatgct gtcgagcgtg tcggccgcgc tcccctgagg      1020
   aagctgtcgc gcaaggagcg cttcatcggc cccgccgccg aggccgccga gaacgacatg      1080
   cccgtcaagt acctcctcga cgccatcgag atgacgttcc gcttccagga cgtcgagggt      1140
   gacgacgagt ccaaggagct ctctaccatc atgtccgaga acacccccga ggacgttgtc      1200
   gccaaggtct gcggcatcaa gccgacggag aagatctacc ctcgcctggt cgaggttgtc      1260
   aagcgtgtgc aggctgacag ccaggaggag taacttgcga tggctcttct ggcgttctag      1320
   ctcggcatca ctcacggcgc tgagtggtga ccggcctact gcatctggct ttgggcttct      1380
   tttgatcttt tacttctact ttttctcttc tacaacttgt gttaagaatg cacgaatgaa      1440
   cggcaaggcg ctgggttccg ggttaataga gcatcgtctc ttttgttttc tatttttttt      1500
   gtactctgct agggcattcc ttgacgggaa caaaaggtgc tcgggatatc ttcatgggaa      1560
   gcttcagtct atagatggac tgagcaggca gcataacggg tcaagatacc acatc           1615
<210> 13
   <211> 397
   <212> PRT
   <213> Trichoderma reesei
<400> 13
   Met Ala Asn Ala Thr Gly Lys Lys Ala Val His Phe Gly Ala Gly Asn
   1               5                   10                  15
   Ile Gly Arg Gly Phe Val Ala Cys Phe Leu His Asn Ser Gly Tyr Glu
               20                  25                  30
   Val Val Phe Ala Asp Val Asn Gly Asp Leu Ile Asp Thr Ile Asn Ser
           35                  40                  45
   Thr Pro Ser Tyr Lys Val Ile Glu Val Gly Ser Glu Gly Thr Ser Glu
       50                  55                  60
   Asn Val Ile Thr Asn Tyr Arg Ala Ile Asn Ser Arg Thr His Glu Glu
   65                  70                  75                  80
   Asp Leu Val Glu Glu Ile Arg Thr Ala Asp Val Val Thr Cys Ser Val
                   85                  90                  95
   Gly Pro Asn Ile Leu Lys Phe Ile Ala Pro Val Ile Ala Lys Gly Ile
               100                 105                 110
   Asp Arg Arg Ser Asp Asp Glu Thr Pro Leu His Val Ile Ala Cys Glu
           115                 120                 125
   Asn Ala Ile Gly Ala Thr Asp Thr Leu Ala Ser Tyr Ile Lys Asp Ala
       130                 135                 140
   Lys Asn Thr Asp Pro Ala Arg Leu Glu Thr His His Leu Arg Ala Arg
   145                 150                 155                 160
   Tyr Ala Asn Ser Ala Ile Asp Arg Ile Val Pro Ala Gln Asp Pro Asn
                   165                 170                 175
   Ala Gly Leu Asp Val Lys Leu Glu Lys Phe Phe Glu Trp Val Val Asp
               180                 185                 190
   Lys Thr Pro Phe Glu Asp Ile Gly Ile Pro Pro Ile Glu Gly Ile Gln
           195                 200                 205
   Trp Val Asp Asn Leu Glu Pro Phe Ile Glu Arg Lys Leu Tyr Thr Val
       210                 215                 220
   Asn Thr Gly His Ala Thr Ala Ala Tyr His Gly Tyr Tyr Arg Arg Lys
   225                 230                 235                 240
   Arg Thr Val Tyr Asp Ala Leu Gln Asp Lys Glu Ile Met Ala Glu Val
                   245                 250                 255
   Arg Gly Ala Leu Thr Glu Thr Lys Asn Leu Leu Val Ala Lys His Gly
               260                 265                 270
   Ile Asp Glu Glu Ala Gln Leu Ala Tyr Met Asn Lys Ile Ile Lys Arg
           275                 280                 285
   Ile Gly Asn Pro His Leu Glu Asp Ala Val Glu Arg Val Gly Arg Ala
       290                 295                 300
   Pro Leu Arg Lys Leu Ser Arg Lys Glu Arg Phe Ile Gly Pro Ala Ala
   305                 310                 315                 320
   Glu Ala Ala Glu Asn Asp Met Pro Val Lys Tyr Leu Leu Asp Ala Ile
                   325                 330                 335
   Glu Met Thr Phe Arg Phe Gln Asp Val Glu Gly Asp Asp Glu Ser Lys
               340                 345                 350
   Glu Leu Ser Thr Ile Met Ser Glu Asn Thr Pro Glu Asp Val Val Ala
   355 360 365
   Lys Val Cys Gly Ile Lys Pro Thr Glu Lys Ile Tyr Pro Arg Leu Val
   370 375 380
   Glu Val Val Lys Arg Val Gln Ala Asp Ser Gln Glu Glu
   385 390 395
<210> 14
   <211> 1418
   <212> DNA
   <213> Trichoderma reesei
<400> 14
   cagacaacta caaacaagat ggttggcgtt gacgcagaga ttatcgccga tgataccaga        60
   atcctgggtc aggatccttt gatccccccg gctctgctca tctccgagat ccccatgact       120
   cccaatgccc tccagaccgt cgtcaagggc cggagagacg ctgcccagat catcatgggc       180
   cagaacgaca ggctattagt cgtcgtcggt ccctgctcca tccacgaccc cgaagccgcc       240
   cacgagtacg ccgcccgcct caagcagctc tccgagaagc tcgccgacga cctctgcatc       300
   gtgatgcgcg cctacctgga gaagcctcgc acgaccgtcg gctggaaggg cctgatcaac       360
   gatcccgata tcgactcgtc cttccagatc aacaagggcc tgcgcgtctc ccgccggctc       420
   ttcgtcgacc tgacctccaa cggcatgccc attgccaccg agatgctcga caccatctcc       480
   ccccagttcc tcgccgactg catctccgtc ggcgccattg gcgcgcgaac gaccgagtcc       540
   cagctgcacc gtgagctcgc ttccggcctg tccttccccg tgggcttcaa gaacggaacc       600
   gacggcagcc ttggcgtcgc cattgatgcc attggcgctg ccgcggccaa gcaccacttc       660
   atgggtgtga cgaagcaggg cctggctgcc attacccgca ccaagggcaa cgagcacggc       720
   ttcgtgatcc tgagaggcgg aagcaagggc cccaactttg acaaggagag cgtccaggcg       780
   gccaagaaga tgctgacgga caagggccag aagctggcca ttatgattga ctgttctcac       840
   ggcaactctc agaagaacca caacaaccag cccaaggttg ccaaggccat tgccgaccag       900
   ctgcgcgagg gcgagcgagc cattgtcggc gtcatgatcg agtccaacat caacgagggc       960
   aaccaaaagg tccctgctga gggtccggcc ggtctcaagc gcggcgttag catcacggat      1020
   gcatgcatca actgggagga cacagtcacg gtcctcgagg accttgccgc tgccgtccgc      1080
   acccgacgcg aggtcaactc gggtgctcct agcgcagagg ccaaggtgag cactctggag      1140
   gaggactaga cgtagtttta ccggaagggg cgtatgtgtg tacgatgaga gagaggaacg      1200
   aaaagcatga gggtcggttt gttgattttg aacattacga tgcgtgagtt tatgcgcgcg      1260
   aatcctggct tcctccctgg ccagcgtaaa taagaaaagt tttagggaga gcattcgaca      1320
   cggatatcga gaaattggcg caccaaagga acatgtatga aggcatatat tgttggctgt      1380
   ccactttgtc acagacggcg cacacgaacc agtttcag                              1418
<210> 15
   <211> 376
   <212> PRT
   <213> Trichoderma reesei
<400> 15
   Met Val Gly Val Asp Ala Glu Ile Ile Ala Asp Asp Thr Arg Ile Leu
   1               5                   10                  15
   Gly Gln Asp Pro Leu Ile Pro Pro Ala Leu Leu Ile Ser Glu Ile Pro
               20                  25                  30
   Met Thr Pro Asn Ala Leu Gln Thr Val Val Lys Gly Arg Arg Asp Ala
           35                  40                  45
   Ala Gln Ile Ile Met Gly Gln Asn Asp Arg Leu Leu Val Val Val Gly
       50                  55                  60
   Pro Cys Ser Ile His Asp Pro Glu Ala Ala His Glu Tyr Ala Ala Arg
   65                  70                  75                  80
   Leu Lys Gln Leu Ser Glu Lys Leu Ala Asp Asp Leu Cys Ile Val Met
                   85                  90                  95
   Arg Ala Tyr Leu Glu Lys Pro Arg Thr Thr Val Gly Trp Lys Gly Leu
               100                 105                 110
   Ile Asn Asp Pro Asp Ile Asp Ser Ser Phe Gln Ile Asn Lys Gly Leu
           115                 120                 125
   Arg Val Ser Arg Arg Leu Phe Val Asp Leu Thr Ser Asn Gly Met Pro
       130                 135                 140
   Ile Ala Thr Glu Met Leu Asp Thr Ile Ser Pro Gln Phe Leu Ala Asp
   145                 150                 155                 160
   Cys Ile Ser Val Gly Ala Ile Gly Ala Arg Thr Thr Glu Ser Gln Leu
                   165                 170                 175
   His Arg Glu Leu Ala Ser Gly Leu Ser Phe Pro Val Gly Phe Lys Asn
               180                 185                 190
   Gly Thr Asp Gly Ser Leu Gly Val Ala Ile Asp Ala Ile Gly Ala Ala
           195                 200                 205
   Ala Ala Lys His His Phe Met Gly Val Thr Lys Gln Gly Leu Ala Ala
       210                 215                 220
   Ile Thr Arg Thr Lys Gly Asn Glu His Gly Phe Val Ile Leu Arg Gly
   225                 230                 235                 240
   Gly Ser Lys Gly Pro Asn Phe Asp Lys Glu Ser Val Gln Ala Ala Lys
                   245                 250                 255
   Lys Met Leu Thr Asp Lys Gly Gln Lys Leu Ala Ile Met Ile Asp Cys
               260                 265                 270
   Ser His Gly Asn Ser Gln Lys Asn His Asn Asn Gln Pro Lys Val Ala
           275                 280                 285
   Lys Ala Ile Ala Asp Gln Leu Arg Glu Gly Glu Arg Ala Ile Val Gly
       290                 295                 300
   Val Met Ile Glu Ser Asn Ile Asn Glu Gly Asn Gln Lys Val Pro Ala
   305                 310                 315                 320
   Glu Gly Pro Ala Gly Leu Lys Arg Gly Val Ser Ile Thr Asp Ala Cys
                   325                 330                 335
   Ile Asn Trp Glu Asp Thr Val Thr Val Leu Glu Asp Leu Ala Ala Ala
               340                 345                 350
   Val Arg Thr Arg Arg Glu Val Asn Ser Gly Ala Pro Ser Ala Glu Ala
           355                 360                 365
   Lys Val Ser Thr Leu Glu Glu Asp
       370                 375
<210> 16
   <211> 3267
   <212> DNA
   <213> Trichoderma reesei
<400> 16
   atgacaacgc tgctcaacaa cccgccggat tcgggtcccc cgccgcgcca gattcgcttc      60
   gtacataacc agggccagcc gccgtccaag cgacgcagga tcaatgcagc gtatgttctt     120
   ctggggcttc tctcgccttg cttcgtctgt cttgccgcgt cacccttggt ggcaaatggc    180
   tgtcctagac agataagtgc aagatggcgc gagcatcaac accatgcggt taccagtgaa     240
   gctataagta tgtagagcta acctcgtctt gcgcgcagat gcttgacctg tcgaagacga     300
   aagacgagat gcgccggcga acgaccgctt tgcaccacat gcactaagaa cgggcaccaa     360
   tgcctcggtt atcccgagga caggagagat ggcgaggcag cggcaaggac gggcgacgag 420
   gaaacgacgc caagggagga tgtcaaccaa ggcgaggacg acgtcgcctc tagaaaaccc 480
   gctgcttcgg ggcttgcgaa ccgtccgacg tcctctgggg gcagcaatct gcaagctacg 540
   catgctgctg cctctgctgc ctctgcttca cggccgccgc ggcgcaatat caagcagact 600
   atcgaagtcg gcgccgatgc caacccgggc gacgcagagc cgcaacagtc tcccgtacta 660
   catcaccacc accagagcca ccaccagaat caccatcctc atcatcaagc tcatctctca 720
   gtcacgtccg atgacttgcc cagttcgccc accctccgcc gatcggcccc cagccgtcgc 780
   atcccctatt tccgctactt tggctccacg gccatcgtcc ccggcttcaa gcaaatggtg 840
   gtctctgttc gcgaccgtcg ccgttcgacg gcaggctcac attccggcgc ctctcttcat 900
   tcgacgccca gcggggcgat ggccagtggc tcggccattg acagcgacat tgtccgcgaa 960
   gacattccct cctacgaccc caacaactcc gcccccgttc atcccttaat cattcagcta 1020
   gtcaatacgt tttttgttca cgtcgggtgc aattacccct ttctcaaaca agacaagttt 1080
   ctgcaacatg taaaggagaa acgggtcgag ccgatacttg tcgatgccgt ctgcgccatc 1140
   gcggcgcgct tctcggacca tcacatcctg acgggcggca acgacaagat gccccggaca 1200
   gagagaggcc aggtctttgc gcaacgggcc aggcaggcca ccgtcgacac tttcccttgc 1260
   ccttctgtgg gtgcggtcca ggcttgcctg ctgatggcat acgagggctt cggggcggcc 1320
   caggatagcg cgctgtggat gtatctgggc ttggccattc gcatggcggt tgatctcggt 1380
   ctccaaaagt gcgtgggcgt tcaatatcaa ggcgaaaagg acccttggta cacccggcat 1440
   cgaagtcggg ccaacgacga tgatcaggac agcccagagg cgcacaaggc ggatgccgtc 1500
   gacgccccta gcccggagga acagaaggag gtagagcagg agcgcatata taccttctgg 1560
   tccgtcttca tcctggaccg gctcatctcc tctggcactg gacggccggt cacgatccgc 1620
   gacggcgact acgagctcgc ctttcccgaa tccagcgtcg acctcgctac cggatggccg 1680
   aacccatttc ctgttctcct ccagatcatc cacctctacg gtcaagtgtg cgatgtgctg 1740
   aacaatctcc acgacgccca agaccttacc caggacaagt gggatcagct atccgagatg 1800
   gagcatcgac tcacgagaat gtacaagcac tgggacccgc ggcttcagtt caacgtcaac 1860
   aactttaaga cgtacctcgg catgggccag ggcaccaact ttatcctcct ccacttttgg 1920
   ttccacgcgc tcttcatcat attgcatcaa ccgactcttc ttaccccctt ttgcgaactg 1980
   cggagcgagc tccagctgct ctcggacagc cgtgagctga gcatgagcag cgcaaagacc 2040
   atctgcgaca ttttgtcctt tgccgacttg atagatccga cgagttttat tggcaaccca 2100
   ttcacgaatc agcccattta tatcgcggca tgcgcatttc tcatggaatc gagcgccaac 2160
   aatgcatctg agggctcgtc cagggagggc tcgcctccca ctcaagcgag tcgctccagg 2220
   caacagacga agcactttaa caagcagtct cggcattcgc ttctcgcgtc ggccgccaac 2280
   cagaattacc agcgatgcta caactccctg cagcagatcc aggcgtactg gggcggagta     2340
   ggctatatcc tcaccgcgct ggaccagaag tcaaagggca agtgggactg cgagacgtat     2400
   accgtggagg agtatgagag caccaagctg ccccctcctc ggccgtcgat tggtgagcag     2460
   ctgtcgcggt tcgagaagca gtcgtcgccc aagattgccg gctcaccgat tgcttggagt     2520
   ctgtccggca cggccaattc gcccaactcc agcctgacac tcatgtatca ggctctcaac     2580
   gccagcgcag gcggccacac tggattccct ccgcctgtgc agtccgtggc gagggctgcc     2640
   aatggttcgc cggggagcat cgcgctggac cccattcgac acacgatgcc ggaagtggga     2700
   atgtttacgt cggcggttcc gcagccaaac atgtctgcca ttcgaaactc tcctcggcgg     2760
   ccatatgctg ccaattctat gaatgccgcg ggggcgaact cgcggaatca gaatatcatg      2820
   ggcttcgaag acattccgga agagtcgccg gatgcaaagg gtctgatgtc caattactca     2880
   acctctggcc agccctacga tggatataat gcatccccga ccagcggcaa ttcgggcatg     2940
   ggcaattcta cagccgtgac gagtgccccc agtggaaaca cgacgtcgaa tgcgaatacc     3000
   cacaacaata acaacaacaa cagcaccacc accaccagca gcagcaacag caacagcatg     3060
   tatttcgcgc aaaacatggc atcatatcaa gcctgggggc cgatgacggg caacatggac     3120
   gccatcacgt tcgacagcca ggacattgac attgaggcgc tggggctgca gcagccggac     3180
   ctgatgagcg ggtggctcga gtatcttccc agtgatgtgc tggggctgtt tgacgagcat     3240
   ggcgagtcgg ggcagggcgg ccattga                                          3267
<210> 17
   <211> 983
   <212> PRT
   <213> Trichoderma reesei
<400> 17
   Met Thr Thr Leu Leu Asn Asn Pro Pro Asp Ser Gly Pro Pro Pro Arg
   1               5                   10                  15
   Gln Ile Arg Phe Val His Asn Gln Gly Gln Pro Pro Ser Lys Arg Arg
               20                  25                  30
   Arg Ile Asn Ala Ala Cys Leu Thr Cys Arg Arg Arg Lys Thr Arg Cys
           35                  40                  45
   Ala Gly Glu Arg Pro Leu Cys Thr Thr Cys Thr Lys Asn Gly His Gln
       50                  55                  60
   Cys Leu Gly Tyr Pro Glu Asp Arg Arg Asp Gly Glu Ala Ala Ala Arg
   65                  70                  75                  80
   Thr Gly Asp Glu Glu Thr Thr Pro Arg Glu Asp Val Asn Gln Gly Glu
                   85                  90                  95
   Asp Asp Val Ala Ser Arg Lys Pro Ala Ala Ser Gly Leu Ala Asn Arg
               100                 105                 110
   Pro Thr Ser Ser Gly Gly Ser Asn Leu Gln Ala Thr His Ala Ala Ala
           115                 120                 125
   Ser Ala Ala Ser Ala Ser Arg Pro Pro Arg Arg Asn Ile Lys Gln Thr
       130                 135                 140
   Ile Glu Asn His His Pro His His Gln Ala His Leu Ser Val Thr Ser
   145                 150                 155                 160
   Asp Asp Leu Pro Ser Ser Pro Thr Leu Arg Arg Ser Ala Pro Ser Arg
                   165                 170                 175
   Arg Ile Pro Tyr Phe Arg Tyr Phe Gly Ser Thr Ala Ile Val Pro Gly
               180                 185                 190
   Phe Lys Gln Met Val Val Ser Val Arg Asp Arg Arg Arg Ser Thr Ala
           195                 200                 205
   Gly Ser His Ser Gly Ala Ser Leu His Ser Thr Pro Ser Gly Ala Met
       210                 215                 220
   Ala Ser Gly Ser Ala Ile Asp Ser Asp Ile Val Arg Glu Asp Ile Pro
   225                 230                 235                 240
   Ser Tyr Asp Pro Asn Asn Ser Ala Pro Val His Pro Leu Ile Ile Gln
                   245                 250                 255
   Leu Val Asn Thr Phe Phe Val His Val Gly Cys Asn Tyr Pro Phe Leu
               260                 265                 270
   Lys Gln Asp Lys Phe Leu Gln His Val Lys Glu Lys Arg Val Glu Pro
           275                 280                 285
   Ile Leu Val Asp Ala Val Cys Ala Ile Ala Ala Arg Phe Ser Asp His
       290                 295                 300
   His Ile Leu Thr Gly Gly Asn Asp Lys Met Pro Arg Thr Glu Arg Gly
   305                 310                 315                 320
   Gln Val Phe Ala Gln Arg Ala Arg Gln Ala Thr Val Asp Thr Phe Pro
                   325                 330                 335
   Cys Pro Ser Val Gly Ala Val Gln Ala Cys Leu Leu Met Ala Tyr Glu
               340                 345                 350
   Gly Phe Gly Ala Ala Gln Asp Ser Ala Leu Trp Met Tyr Leu Gly Leu
           355                 360                 365
   Ala Ile Arg Met Ala Val Asp Leu Gly Leu Gln Lys Cys Val Gly Val
       370                 375                 380
   Gln Tyr Gln Gly Glu Lys Asp Pro Trp Tyr Thr Arg His Arg Ser Arg
   385                 390                 395                 400
   Ala Asn Asp Asp Asp Gln Asp Ser Pro Glu Ala His Lys Ala Asp Ala
                   405                 410                 415
   Val Asp Ala Pro Ser Pro Glu Glu Gln Lys Glu Val Glu Gln Glu Arg
               420                 425                 430
   Ile Tyr Thr Phe Trp Ser Val Phe Ile Leu Asp Arg Leu Ile Ser Ser
           435                 440                 445
   Gly Thr Gly Arg Pro Val Thr Ile Arg Asp Gly Asp Tyr Glu Leu Ala
       450                 455                 460
   Phe Pro Glu Ser Ser Val Asp Leu Ala Thr Gly Trp Pro Asn Pro Phe
   465                 470                 475                 480
   Pro Val Leu Leu Gln Ile Ile His Leu Tyr Gly Gln Val Cys Asp Val
                   485                 490                 495
   Leu Asn Asn Leu His Asp Ala Gln Asp Leu Thr Gln Asp Lys Trp Asp
               500                 505                 510
   Gln Leu Ser Glu Met Glu His Arg Leu Thr Arg Met Tyr Lys His Trp
           515                 520                 525
   Asp Pro Arg Leu Gln Phe Asn Val Asn Asn Phe Lys Thr Tyr Leu Gly
       530                 535                 540
   Met Gly Gln Gly Thr Asn Phe Ile Leu Leu His Phe Trp Phe His Ala
   545                 550                 555                 560
   Leu Phe Ile Ile Leu His Gln Pro Thr Leu Leu Thr Pro Phe Cys Glu
                   565                 570                 575
   Leu Arg Ser Glu Leu Gln Leu Leu Ser Asp Ser Arg Glu Leu Ser Met
               580                 585                 590
   Ser Ser Ala Lys Thr Ile Cys Asp Ile Leu Ser Phe Ala Asp Leu Ile
           595                 600                 605
   Asp Pro Thr Ser Phe Ile Gly Asn Pro Phe Thr Asn Gln Pro Ile Tyr
       610                 615                 620
   Ile Ala Ala Cys Ala Phe Leu Met Glu Ser Ser Ala Asn Asn Ala Ser
   625                 630                 635                 640
   Glu Gly Ser Ser Arg Glu Gly Ser Pro Pro Thr Gln Ala Ser Arg Ser
                   645                 650                 655
   Arg Gln Gln Thr Lys His Phe Asn Lys Gln Ser Arg His Ser Leu Leu
               660                 665                 670
   Ala Ser Ala Ala Asn Gln Asn Tyr Gln Arg Cys Tyr Asn Ser Leu Gln
           675                 680                 685
   Gln Ile Gln Ala Tyr Trp Gly Gly Val Gly Tyr Ile Leu Thr Ala Leu
       690                 695                 700
   Asp Gln Lys Ser Lys Gly Lys Trp Asp Cys Glu Thr Tyr Thr Val Glu
   705                 710                 715                 720
   Glu Tyr Glu Ser Thr Lys Leu Pro Pro Pro Arg Pro Ser Ile Gly Glu
                   725                 730                 735
   Gln Leu Ser Arg Phe Glu Lys Gln Ser Ser Pro Lys Ile Ala Gly Ser
               740                 745                 750
   Pro Ile Ala Trp Ser Leu Ser Gly Thr Ala Asn Ser Pro Asn Ser Ser
           755                 760                 765
   Leu Thr Leu Met Tyr Gln Ala Leu Asn Ala Ser Ala Gly Gly His Thr
       770                 775                 780
   Gly Phe Pro Pro Pro Val Gln Ser Val Ala Arg Ala Ala Asn Gly Ser
   785                 790                 795                 800
   Pro Gly Ser Ile Ala Leu Asp Pro Ile Arg His Thr Met Pro Glu Val
                   805                 810                 815
   Gly Met Phe Thr Ser Ala Val Pro Gln Pro Asn Met Ser Ala Ile Arg
               820                 825                 830
   Asn Ser Pro Arg Arg Pro Tyr Ala Ala Asn Ser Met Asn Ala Ala Gly
           835                 840                 845
   Ala Asn Ser Arg Asn Gln Asn Ile Met Gly Phe Glu Asp Ile Pro Glu
       850                 855                 860
   Glu Ser Pro Asp Ala Lys Gly Leu Met Ser Asn Tyr Ser Thr Ser Gly
   865                 870                 875                 880
   Gln Pro Tyr Asp Gly Tyr Asn Ala Ser Pro Thr Ser Gly Asn Ser Gly
                   885                 890                 895
   Met Gly Asn Ser Thr Ala Val Thr Ser Ala Pro Ser Gly Asn Thr Thr
               900                 905                 910
   Asn Ser Met Tyr Phe Ala Gln Asn Met Ala Ser Tyr Gln Ala Trp Gly
           915                 920                 925
   Pro Met Thr Gly Asn Met Asp Ala Ile Thr Phe Asp Ser Gln Asp Ile
       930                 935                 940
   Asp Ile Glu Ala Leu Gly Leu Gln Gln Pro Asp Leu Met Ser Gly Trp
   945                 950                 955                 960
   Leu Glu Tyr Leu Pro Ser Asp Val Leu Gly Leu Phe Asp Glu His Gly
                   965                 970                 975
   Glu Ser Gly Gln Gly Gly His
               980
<210> 18
   <211> 1773
   <212> DNA
   <213> Trichoderma reesei
<400> 18
   atgtcaacca gacacatttt tacgtcatgt gacggcctcg tcaacaaggc catccgaggc      60
   atcattacct acaacccttc tctcagccta gacgaaagca accgggtcgt ctatgacacc     120
   acatacgaca agtccaaagt cgtcatcata agcggaggcg gctccggaca tgagcctgcg     180
   tggacaggct atgtcggtca aaacatgctc gcagcttcgg ttcaaggaga catctttgcc     240
   agccccagca caaagcaaat ccttgcagca gtggacgctg ttccgagtga caagggcacc     300
   attctggtca tcacaaacta cactggtgac tgtctgcact ttgggctggc caacgagaaa     360
   gccaatgcca aaggccacaa ctgccggacc atcatctgcg gcgacgatgt ctctgtcggc     420
   aagaacggga gcatggttgg tcgcagaggt ttggctggac agatgggtgt cctcaaagtc     480
   atgggcggag ctgctggtgc tggaggctcg ctggacgaag tttacaatct tggtgtcgca     540
   ttctcgcagc agattgtgtc tattgcggca accttggatc attgccatgt tccaggacgt     600
   gctgaacatg gcatgcttca agaaggcgtt gttgaactcg gcaccggccc tcataacgaa     660
   cccggataca agaaactatc ccctgcgcca tctgcgtctg atctcgtctc ccagattcta     720
   caatactgtc tgaatgaaaa ggatccggaa agaggctacg ttcacttcgc ccctagtgat     780
   gaagtcatac tacttctcag caactacggc ggcatatcgc acctggagat gggcgctctg     840
   gttgacgaaa ttctcgagca gcttgcaaga gactggaaga ttgagccggt gagggtatgc     900
   gccggcttca tcgagacgtc actcaatgct ccagcctttt ctgtctccgt catcaatgtg     960
   actgctgccg caaagaattg cagttactct gtcgaagaga tcaagtcctt ctttgatgcc    1020
   aggacgacga cgttttggga gtccatgact gggtcgcaga cacggcggag gacgagacag    1080
   cagcagattg ttcagccccc ggaggaagag gtcaaggcag ttgatgagtc aaaagatctg    1140
   actattgatc ccacgctctt agagaagatg ttgaggaacg cttgtgaagc gctcgtcaag    1200
   gctgagccag atctcaccaa gtgggatacc gtcatgggtg atggagattg cggagagacg    1260
   ctcaggactg gagccacatc tttgctcacg gcccttgatt cgggattggc gaaatctggg    1320
   tcagttgtga gagtactatt ggaactcgaa gacatcgtgg agagcaagat gggcggtact    1380
   ctgggcggca tattgggcat cttttttgtc tccttgcgtg cagctgtcga gaagaatatt     1440
   ggactggccc aaagcccggt cttgctctgg ggaacagctc ttgcaacagc tctggagagt    1500
   ctcaggaggt atacgcctgc caaggtcgga gatcgtactg tcatggacac actcatcccg    1560
   tttgctaccg tcatgtgtga gtcgggcttt gataacggcg tcaaggcggc acaggatgga    1620
   gcggatgcga ccaagacgat gaagccgaga ctgggaagag caacgtatgt tggcgtgtcg    1680
   agtgagaaca aggagttgcc tcctgatccc ggtgcttggg gggccatggt ggccattcgc    1740
   ggtttgcgac aaggtctggt tgaagcgatc tag                                 1773
<210> 19
   <211> 590
   <212> PRT
   <213> Trichoderma reesei
<400> 19
   Met Ser Thr Arg His Ile Phe Thr Ser Cys Asp Gly Leu Val Asn Lys
   1               5                   10                  15
   Ala Ile Arg Gly Ile Ile Thr Tyr Asn Pro Ser Leu Ser Leu Asp Glu
               20                  25                  30
   Ser Asn Arg Val Val Tyr Asp Thr Thr Tyr Asp Lys Ser Lys Val Val
           35                  40                  45
   Ile Ile Ser Gly Gly Gly Ser Gly His Glu Pro Ala Trp Thr Gly Tyr
       50                  55                  60
   Val Gly Gln Asn Met Leu Ala Ala Ser Val Gln Gly Asp Ile Phe Ala
   65                  70                  75                  80
   Ser Pro Ser Thr Lys Gln Ile Leu Ala Ala Val Asp Ala Val Pro Ser
                   85                  90                  95
   Asp Lys Gly Thr Ile Leu Val Ile Thr Asn Tyr Thr Gly Asp Cys Leu
               100                 105                 110
   His Phe Gly Leu Ala Asn Glu Lys Ala Asn Ala Lys Gly His Asn Cys
           115                 120                 125
   Arg Thr Ile Ile Cys Gly Asp Asp Val Ser Val Gly Lys Asn Gly Ser
       130                 135                 140
   Met Val Gly Arg Arg Gly Leu Ala Gly Gln Met Gly Val Leu Lys Val
   145                 150                 155                 160
   Met Gly Gly Ala Ala Gly Ala Gly Gly Ser Leu Asp Glu Val Tyr Asn
                   165                 170                 175
   Leu Gly Val Ala Phe Ser Gln Gln Ile Val Ser Ile Ala Ala Thr Leu
               180                 185                 190
   Asp His Cys His Val Pro Gly Arg Ala Glu His Gly Met Leu Gln Glu
           195                 200                 205
   Gly Val Val Glu Leu Gly Thr Gly Pro His Asn Glu Pro Gly Tyr Lys
       210                 215                 220
   Lys Leu Ser Pro Ala Pro Ser Ala Ser Asp Leu Val Ser Gln Ile Leu
   225                 230                 235                 240
   Gln Tyr Cys Leu Asn Glu Lys Asp Pro Glu Arg Gly Tyr Val His Phe
                   245                 250                 255
   Ala Pro Ser Asp Glu Val Ile Leu Leu Leu Ser Asn Tyr Gly Gly Ile
               260                 265                 270
   Ser His Leu Glu Met Gly Ala Leu Val Asp Glu Ile Leu Glu Gln Leu
           275                 280                 285
   Ala Arg Asp Trp Lys Ile Glu Pro Val Arg Val Cys Ala Gly Phe Ile
       290                 295                 300
   Glu Thr Ser Leu Asn Ala Pro Ala Phe Ser Val Ser Val Ile Asn Val
   305 310 315 320
   Thr Ala Ala Ala Lys Asn Cys Ser Tyr Ser Val Glu Glu Ile Lys Ser
   325 330 335
   Phe Phe Asp Ala Arg Thr Thr Thr Phe Trp Glu Ser Met Thr Gly Ser
   340 345 350
   Gln Thr Arg Arg Arg Thr Arg Gln Gln Gln Ile Val Gln Pro Pro Glu
   355 360 365
   Glu Glu Val Lys Ala Val Asp Glu Ser Lys Asp Leu Thr Ile Asp Pro
   370 375 380
   Thr Leu Leu Glu Lys Met Leu Arg Asn Ala Cys Glu Ala Leu Val Lys
   385 390 395 400
   Ala Glu Pro Asp Leu Thr Lys Trp Asp Thr Val Met Gly Asp Gly Asp
   405 410 415
   Cys Gly Glu Thr Leu Arg Thr Gly Ala Thr Ser Leu Leu Thr Ala Leu
   420 425 430
   Asp Ser Gly Leu Ala Lys Ser Gly Ser Val Val Arg Val Leu Leu Glu
   435 440 445
   Leu Glu Asp Ile Val Glu Ser Lys Met Gly Gly Thr Leu Gly Gly Ile
   450 455 460
   Leu Gly Ile Phe Phe Val Ser Leu Arg Ala Ala Val Glu Lys Asn Ile
   465 470 475 480
   Gly Leu Ala Gln Ser Pro Val Leu Leu Trp Gly Thr Ala Leu Ala Thr
   485 490 495
   Ala Leu Glu Ser Leu Arg Arg Tyr Thr Pro Ala Lys Val Gly Asp Arg
   500 505 510
   Thr Val Met Asp Thr Leu Ile Pro Phe Ala Thr Val Met Cys Glu Ser
   515 520 525
   Gly Phe Asp Asn Gly Val Lys Ala Ala Gln Asp Gly Ala Asp Ala Thr
   530 535 540
   Lys Thr Met Lys Pro Arg Leu Gly Arg Ala Thr Tyr Val Gly Val Ser
   545 550 555 560
   Ser Glu Asn Lys Glu Leu Pro Pro Asp Pro Gly Ala Trp Gly Ala Met
                   565                 570                 575
   Val Ala Ile Arg Gly Leu Arg Gln Gly Leu Val Glu Ala Ile
               580                 585                 590
<210> 20
   <211> 1326
   <212> DNA
   <213> Trichoderma reesei
<400> 20
   ttaggtgagg gtgtaaccgc catcaacgcg aatatcggca ccggtcacat agcggctggc      60
   atcagacagc aggaaagcca ctgggcccat gagatcctga ggcagaccca tcttgccctg     120
   aggaatgagg gacacccatt tcttcttcag gtcaggcttt tcatccagga ttttctcggt     180
   cctagttaag gatttgagtt agccggccga ggtcacggct accgttcaag aacagagaag     240
   agtgggttca actcacaaag ccgtcagcat gtatcccggc gagatacagt tgactcggat     300
   gccagcatgc gcccactcaa cggcgagcga agccgcgaga tggcggactg cggccttgga     360
   ggcgttatag ggcctagtcc agttcaaccc gttagctgtc ttatgctctg ctcatccatg     420
   tctccttttt ttctttcttt cttttttccg tcgtggaagg gactcacgct tgaggctggg      480
   ggacgttgac aatggccccc gacatgctgc ctatcatgac catgctacca gggctcttcc     540
   tctccatgag gtgacgagca accgatgtag cgaagagata tgtgccgtcg acgttgacgc     600
   cccagagctt gcgaactcta tcaatggggt agttggtggc ctcgaagttc tcggtgaagc     660
   cagccgaggt gaccaggttg tcaatcttgc catgttgctg gatgatctcg gcgatacacg     720
   cctcaacgga atcggcatct gagacgtcgg cgtaatgcgc cgtcactttg ggaacgctag     780
   accgggtgtc agccgtctgc ctgcaaagcc gtgttacgtg cagagcggag cggcagctca     840
   ctcaccgttt tgcgttgggg ttctccttga gaaaggcatc cacgagcagt ttcgtctgct     900
   tctcggcctc ctcctctgtc gctgcgtcag ctgtctggcc tggggtacga gcgacccgga     960
   actggcacgc ggaatctcac tgttcatgtc gacgagggcc aggtcggcgc cagagaagac    1020
   cattccctgg ccaatcacca gtcccagccc tcttgcgccg ccggtgacga caccgacgtt    1080
   tccatcgagg gagaagccgg caagcgtagg cttgacatac tggccgccgg tgccagagac    1140
   gggctcgctg ctcggaagct catgctccgg aggatactcg aactcgacct cggcgtcggt    1200
   tcgagagtgg ctgccgggct tatcctgccg cggcggcggc acactatgga acttcctcgc    1260
   agcggccagc ggcaggcgtc tggcgggcgc ggaggcaaag gttcggcgag ccgcaagccg    1320
   cagcat                                                               1326
<210> 21
   <211> 334
   <212> PRT
   <213> Trichoderma reesei
<400> 21
   Met Leu Arg Leu Ala Ala Arg Arg Thr Phe Ala Ser Ala Pro Ala Arg
   1               5                   10                  15
   Arg Leu Pro Leu Ala Ala Ala Arg Lys Phe His Ser Val Pro Pro Pro
               20                  25                  30
   Arg Gln Asp Lys Pro Gly Ser His Ser Arg Thr Asp Ala Glu Val Glu
           35                  40                  45
   Phe Glu Tyr Pro Pro Glu His Glu Leu Pro Ser Ser Glu Pro Val Ser
       50                  55                  60
   Gly Thr Gly Gly Gln Tyr Val Lys Pro Thr Leu Ala Gly Phe Ser Leu
   65                  70                  75                  80
   Asp Gly Asn Val Gly Val Val Thr Gly Gly Ala Arg Gly Leu Gly Leu
                   85                  90                  95
   Val Ile Gly Gln Gly Met Val Phe Ser Gly Ala Asp Leu Ala Leu Val
               100                 105                 110
   Asp Met Asn Ser Glu Ile Pro Arg Ala Ser Ser Gly Ser Leu Thr Ala
           115                 120                 125
   Asp Thr Arg Ser Ser Val Pro Lys Val Thr Ala His Tyr Ala Asp Val
       130                 135                 140
   Ser Asp Ala Asp Ser Val Glu Ala Cys Ile Ala Glu Ile Ile Gln Gln
   145                 150                 155                 160
   His Gly Lys Ile Asp Asn Leu Val Thr Ser Ala Gly Phe Thr Glu Asn
                   165                 170                 175
   Phe Glu Ala Thr Asn Tyr Pro Ile Asp Arg Val Arg Lys Leu Trp Gly
               180                 185                 190
   Val Asn Val Asp Gly Thr Tyr Leu Phe Ala Thr Ser Val Ala Arg His
           195                 200                 205
   Leu Met Glu Arg Lys Ser Pro Gly Ser Met Val Met Ile Gly Ser Met
       210                 215                 220
   Ser Gly Ala Ile Val Asn Val Pro Gln Pro Gln Ala Pro Tyr Asn Ala
   225 230 235 240
   Ser Lys Ala Ala Val Arg His Leu Ala Ala Ser Leu Ala Val Glu Trp
   245 250 255
   Ala His Ala Gly Ile Arg Val Asn Cys Ile Ser Pro Gly Tyr Met Leu
   260 265 270
   Thr Ala Leu Thr Glu Lys Ile Leu Asp Glu Lys Pro Asp Leu Lys Lys
   275 280 285
   Lys Trp Val Ser Leu Ile Pro Gln Gly Lys Met Gly Leu Pro Gln Asp
   290 295 300
   Leu Met Gly Pro Val Ala Phe Leu Leu Ser Asp Ala Ser Arg Tyr Val
   305 310 315 320
   Thr Gly Ala Asp Ile Arg Val Asp Gly Gly Tyr Thr Leu Thr
   325 330
<210> 22
   <211> 640
   <212> DNA
   <213> Trichoderma reesei
<400> 22
   tcatgcctca agatgcccaa tctcttccac cttcttcgca ctcgcactcg atggatcaaa 60
   ctcatacccc agccactcag tagccaacct cctcgcaact tccagcccaa caaccctctc 120
   ccccatacac aacacctgcg cattattgct ctttacactg cgctcaacgc taaagctatc 180
   atgtgccgta accgctcgta tgcccttgac cttgttggca gaaatggcga cgcctagacc 240
   tgtgccgcag atgagcagag cgcggtccgc tttgccctcg gcgacgaggc gggcggcggc 300
   ggcggcgcgc tggggatagg gtgtcttgtc ggagggctcg tcggatccta cgtcgatgac 360
   ttggtctacg cgggcgtcgc tttcaaagtc ttttttgatg gtgtttttgt agctgacgcc 420
   ggcgtcctgc ggaagcgtga gtgagctcgt gtcagtgact gcccgttctg atgacctaga 480
   ttatgcaatc ttgacgatga agagaccgat agtcatgaag acttatgaag ttgacacttg 540
   atctcgttgt gatatgagat gtgctggttc tggcagccga aacagaagca cttacatcgc 600
   agcccatggc gattctccat ttcggtgcca tcgtatatgt 640
<210> 23
   <211> 156
   <212> PRT
   <213> Trichoderma reesei
<400> 23
   Thr Tyr Thr Met Ala Pro Lys Trp Arg Ile Ala Met Gly Cys Asp Asp
   1               5                   10                  15
   Ala Gly Val Ser Tyr Lys Asn Thr Ile Lys Lys Asp Phe Glu Ser Asp
               20                  25                  30
   Ala Arg Val Asp Gln Val Ile Asp Val Gly Ser Asp Glu Pro Ser Asp
           35                  40                  45
   Lys Thr Pro Tyr Pro Gln Arg Ala Ala Ala Ala Ala Arg Leu Val Ala
       50                  55                  60
   Glu Gly Lys Ala Asp Arg Ala Leu Leu Ile Cys Gly Thr Gly Leu Gly
   65                  70                  75                  80
   Val Ala Ile Ser Ala Asn Lys Val Lys Gly Ile Arg Ala Val Thr Ala
                   85                  90                  95
   His Asp Ser Phe Ser Val Glu Arg Ser Val Lys Ser Asn Asn Ala Gln
               100                 105                 110
   Val Leu Cys Met Gly Glu Arg Val Val Gly Leu Glu Val Ala Arg Arg
           115                 120                 125
   Leu Ala Thr Glu Trp Leu Gly Tyr Glu Phe Asp Pro Ser Ser Ala Ser
       130                 135                 140
   Ala Lys Lys Val Glu Glu Ile Gly His Leu Glu Ala
   145                 150                 155
<210> 24
   <211> 726
   <212> DNA
   <213> Trichoderma reesei
<400> 24
   tcacgcttca gctacttcct gtatcgtctt gacaaactgc tccggatcgt gtccgaaccg        60
   ccccaggaac aatccgtcca gtccgctctt caacttctcg tagagccccg gcccggcact       120
   ccccccatac aaaaccctcg tcgtccccgc cctcctccca acggactcca accctctgat       180
   cccagcaaca acatcgagga tgtgcttctc tccagcaggt tgactggcac caatcgccca       240
   tacaggctca tacgccaaaa tgacctctgc ctcggcgggc acagcattca tgacagcatc       300
   aacctgcacc cgacactcct caaccgcgat atgcacgtcg ccttgagtct tttctccaat       360
   gcacacaagc ggtatcatgc cattgcgact tacagcggca gccttattcg caaccatggt       420
   gtcgtcctcg ccaaagatgc tcctgcgctc tgcatgtccg acttccacga gcttgacgcc       480
   tgcgctgcgc aacacggagg ggctgacttc gccggtgaat gcgccttggt cttcccagtg       540
   gcagtcttga gcgccggtcc aaatggcaac ggagctggac ttaagctggg cgattgtggt       600
   ggtcagggag acgaagtcgg ggatgatgaa gatgtcaatg tgggagagga gctctatcgg       660
   gacgtctgcc agcttggata agaagctgtt tgtgaagtct cttgtttgct ggagagaaaa       720
   gtacat                                                                  726
<210> 25
   <211> 241
   <212> PRT
   <213> Trichoderma reesei
<400> 25
   Met Tyr Phe Ser Leu Gln Gln Thr Arg Asp Phe Thr Asn Ser Phe Leu
   1               5                   10                  15
   Ser Lys Leu Ala Asp Val Pro Ile Glu Leu Leu Ser His Ile Asp Ile
               20                  25                  30
   Phe Ile Ile Pro Asp Phe Val Ser Leu Thr Thr Thr Ile Ala Gln Leu
           35                  40                  45
   Lys Ser Ser Ser Val Ala Ile Trp Thr Gly Ala Gln Asp Cys His Trp
       50                  55                  60
   Glu Asp Gln Gly Ala Phe Thr Gly Glu Val Ser Pro Ser Val Leu Arg
   65                  70                  75                  80
   Ser Ala Gly Val Lys Leu Val Glu Val Gly His Ala Glu Arg Arg Ser
                   85                  90                  95
   Ile Phe Gly Glu Asp Asp Thr Met Val Ala Asn Lys Ala Ala Ala Val
               100                 105                 110
   Ser Arg Asn Gly Met Ile Pro Leu Val Cys Ile Gly Glu Lys Thr Gln
           115                 120                 125
   Gly Asp Val His Ile Ala Val Glu Glu Cys Arg Val Gln Val Asp Ala
       130                 135                 140
   Val Met Asn Ala Val Pro Ala Glu Ala Glu Val Ile Leu Ala Tyr Glu
   145                 150                 155                 160
   Pro Val Trp Ala Ile Gly Ala Ser Gln Pro Ala Gly Glu Lys His Ile
                   165                 170                 175
   Leu Asp Val Val Ala Gly Ile Arg Gly Leu Glu Ser Val Gly Arg Arg
               180                 185                 190
   Ala Gly Thr Thr Arg Val Leu Tyr Gly Gly Ser Ala Gly Pro Gly Leu
           195                 200                 205
   Tyr Glu Lys Leu Lys Ser Gly Leu Asp Gly Leu Phe Leu Gly Arg Phe
       210                 215                 220
   Gly His Asp Pro Glu Gln Phe Val Lys Thr Ile Gln Glu Val Ala Glu
   225                 230                 235                 240
   Ala

## Description

The present invention pertains to a genetically modified saprotroph for the biotechnological production of erythritol and a method for the production of erythritol using said genetically modified saprotroph.

In the recent years people's lifestyle and the growing consumption of food products with high sugar content has resulted in a tremendous rise of blood glucose related diseases and disorders, such as diabetes mellitus type 2 (DMT2). Nowadays, a low-glycaemic nutrition and the avoidance of excessive peaks in blood glucose level is considered to reduce the risk for developing certain chronic diseases and to be beneficial for maintenance and improvement of health and for the treatment and/or prevention of a large number of blood glucose related diseases and disorders.

Erythritol is a naturally occurring four-carbon sugar alcohol gaining increasing importance in the food industry due to its specific properties and its manifold fields of application. It can be found in several fruits, such as pears, grapes and melons, mushrooms, alcoholic drinks (beer, wine, sake) and fermented food products, such as soy sauce and miso bean paste, but naturally also occurs in biofluids of humans and animals such as eye lens tissue, serum, plasma, fetal fluid and urine. Due to its small molecular weight, erythritol is easily absorbed already in the upper intestine and therefore causes less digestive distress than other sugar alcohols used in the food industry. The majority of ingested erythritol is not metabolized in the human body and is excreted unmodified into the urine without changing blood glucose and insulin levels (Regnat et al., Erythritol as sweetener-wherefrom and whereto?, 2018, Applied Microbiology and Biotechnology, Vol. 102). Furthermore, erythritol is non-cariogenic, thermally stable, crystalizes well and is less hygroscopic than sucrose. Due to the negative enthalpy of dissolution, the consumption of erythritol causes a cooling sensation in the oral cavity. A 10% (w/v) solution of erythritol has 60-80% of the sweetness of sucrose.

However, in contrast to other sugar alcohols, such as sorbitol, xylitol, mannitol, lactitol, and maltitol, which are well-established as sugar alternatives for many years, so far erythritol cannot be chemically produced in a commercially worthwhile way. The production of erythritol from dialdehyde starch using a nickel catalyst at high temperatures results in unsatisfying low yields (Moon et al. Biotechnological production of erythritol and its applications, 2010, Appl. Microbiol. Biotechnol., Vol. 86).

In yeast and fungus species, erythritol is produced via the so-called pentose phosphate pathway. It is synthesized from D-erythrose-4-phosphate through dephosphorylation and subsequent reduction of erythrose. Based thereon, the suitability of osmophilic yeast, such as *Aureobasidium* sp., *Trichosporonoides* sp. and *Candida magnoliae,* for the biotechnological production of erythritol has been investigated in several studies (Ishizuka et al., Breeding of a mutant of Aureobasidium sp. with high erythritol production, 1989, J. Ferm. Bioeng., Vol. 68(5); Ishizuka et al., Purification and Some Properties of an Erythrose Reductase from an Aureobasidium sp. Mutant, 1992, Biosci. Biotech. Biochem., Vol. 56(6); US4939091A; US5962287 A; Oh et al., Increased erythritol production in fedbatch cultures of Torula sp. by controlling glucose concentration, 2001, JIM&B, Vol. 26; Koh et al., Scale-up of erythritol production by an osmophilic mutant of Candida magnoliae, 2003, Biotechnol. Lett., Vol. 25; Ryu et al., Optimization of erythritol production by Candida magnoliae in fed-batch culture, 2000, JIM&B, Vol. 25). More recent studies examined the potential of filamentous fungi to produce erythritol (Jovanovic et al., 2013). EP 2 436 772 A1 discloses the production of erythritol by reduction of erythrose to erythritol using an erythrose reductase from *Hypocrea*, *Gibberella, Aspergillus* or *Penicillum.* In Moon et al. (Biotechnological production of erythritol and its applications, 2010, Appl. Microbiol. Biotechnol., Vol. 86) several ways of improving the biotechnological production of erythritol are discussed including strain improvement, control of substrate concentration, optimization of carbon sources, supplementation of vitamins and minerals and removal of inhibitors and by-products. However, the yields of erythritol obtained from the different strains was unsatisfactory for industrial scale production. A study of Vélëz et al. (Mannitol metabolism in the phytopathogenic fungus Alternaria alternata, 2007, Fungal Genetics and Biology, Vol. 44) investigated the influence of mannitol 1-phosphate 5-dehydrogenase gene disruption on the production of mannitol in *Alternaria alternata.*

Accordingly, there is still a need for a biotechnological method for the production of erythritol with increased yield. The present invention overcomes the disadvantages of the methods in the prior art by the subject-matter of the independent claims, in particular by the genetically modified saprotroph according to the present invention and the method for the production of erythritol according to the present invention.

The present invention in particular pertains to a genetically modified saprotroph comprising at least one gene encoding at least one membrane-bound alditol transporter, at least one gene encoding at least one erythrose reductase and at least one inactivated gene encoding mannitol 1-phosphate 5-dehydrogenase, wherein the saprotroph is a filamentous fungus selected from the genera *Hypocrea, Gibberella, Aspergillus* and *Penicillium.*

Preferably, the saprotroph is a filamentous fungus selected from *Hypocrea jecorina (Trichoderma reesei), Gibberella zeae, Aspergillus niger, Aspergillus nidulans, Aspergillus oryzae* and *Penicillium chrysogenum.*

In a particularly preferred embodiment of the present invention, the genetically modified saprotroph is *Hypocrea jecorina (Trichoderma reesei).* Preferably, the genetically modified saprotroph is based on the *Trichoderma reesei* strain QM6aΔ*tmus53.*

In a further preferred embodiment of the present invention, the at least one gene encoding at least one membrane-bound alditol transporter is *fps1*, in particular codon-optimized *fps1*. Preferably, the at least one gene encoding at least one membrane-bound alditol transporter is *fps1* from *Saccharomyces cerevisiae,* in particular codon-optimized *fps1* from *Saccharomyces cerevisiae.* In a further preferred embodiment, the at least one gene encoding at least one membrane-bound alditol transporter comprises the nucleotide sequence of **SEQ ID No. 1**, in particular consists of the nucleotide sequence of **SEQ ID No. 1.** Preferably, the membrane-bound alditol transporter comprises the amino acid sequence of **SEQ ID No. 2**, in particular consists of the amino acid sequence of **SEQ ID No. 2.**

In a preferred embodiment of the present invention, the at least one gene encoding at least one erythrose reductase is *err1*, in particular codon-optimized *err1.* Preferably, the at least one gene encoding at least one erythrose reductase is *err1*, in particular codon-optimized *err1*, from *Trichoderma reesei, Aspergillus niger* or *Fusarium graminearum.* In a preferred embodiment, the at least one gene encoding at least one erythrose reductase comprises the nucleotide sequence of **SEQ ID No. 3**, in particular consist of the nucleotide sequence of **SEQ ID No. 3.** Preferably, the erythrose reductase comprises the amino acid sequence of **SEQ ID No. 4**, in particular consists of the amino acid sequence of **SEQ ID No. 4.**

In a preferred embodiment of the present invention, the at least one inactivated gene encoding mannitol 1-phosphate 5-dehydrogenase is *mpdh.* Preferably, the at least one gene encoding mannitol 1-phosphate 5-dehydrogenase of the genetically modified saprotroph comprised the nucleotide sequence of **SEQ ID No. 12** before inactivation, in particular consisted of the nucleotide sequence of **SEQ ID No. 12** before inactivation. Preferably, the mannitol 1-phosphate 5-dehydrogenase encoded by the nucleotide sequence of **SEQ ID No. 12** before inactivation comprises the amino acid sequence of **SEQ ID No. 13**, in particular consists of the amino acid sequence of **SEQ ID No. 13.**

In a further preferred embodiment of the present invention, the at least one inactivated gene encoding mannitol 1-phosphate 5-dehydrogenase of the genetically modified saprotroph is deleted.

In a preferred embodiment of the present invention, the at least one inactivated gene encoding mannitol 1-phosphate 5-dehydrogenase of the genetically modified saprotroph is non-functional.

In a particularly preferred embodiment, the at least one inactivated gene encoding mannitol 1-phosphate 5-dehydrogenase of the genetically modified saprotroph is inactivated by gene knock-out, in particular gene replacement. Preferably, the at least one inactivated gene encoding mannitol 1-phosphate 5-dehydrogenase of the genetically modified saprotroph is inactivated by gene replacement using a deletion cassette.

In a preferred embodiment of the present invention, the at least one gene encoding mannitol 1-phosphate 5-dehydrogenase of the genetically modified saprotroph is inactivated, in particular made non-functional, by genome editing, in particular by meganucleases, zinc-finger nucleases (ZFNs), transcription activator-like effector nucleases (TALENs) or by the clustered regularly interspaced short palindromic repeats (CRISPR) system.

In a particularly preferred embodiment, the genetically modified saprotroph comprises at least one, preferably at least two, preferably at least three, preferably at least four, preferably at least five, further inactivated genes. Preferably, the at least one further inactivated gene is selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase 1 (EYI1) and a gene encoding erythritol isomerase 2 (EYI2).

In a preferred embodiment of the present invention, the at least one further inactivated gene is a gene encoding phospho-2-dehydro-3-deoxyheptonate aldolase 1 (Dhaps-1). Preferably, the at least one gene encoding phospho-2-dehydro-3-deoxyheptonate aldolase 1 (Dhaps1) of the genetically modified saprotroph comprised the nucleotide sequence of **SEQ ID No. 14** before inactivation, in particular consisted of the nucleotide sequence of **SEQ ID No. 14** before inactivation. Preferably, the phospho-2-dehydro-3-deoxyheptonate aldolase 1 (Dhaps1) encoded by the nucleotide sequence of **SEQ ID No. 14** before inactivation comprises the amino acid sequence of **SEQ ID No. 15**, in particular consists of the amino acid sequence of **SEQ ID No. 15.**

In another embodiment of the present invention, the at least one further inactivated gene is a gene encoding erythritol utilization factor (EUF). Preferably, the at least one gene encoding erythritol utilization factor (Euf1) of the genetically modified saprotroph comprised the nucleotide sequence of **SEQ ID No. 16** before inactivation, in particular consisted of the nucleotide sequence of **SEQ ID No. 16** before inactivation. Preferably, the erythritol utilization factor (Euf1) encoded by the nucleotide sequence of **SEQ ID No. 16** before inactivation comprises the amino acid sequence of **SEQ ID No. 17**, in particular consists of the amino acid sequence of **SEQ ID No. 17.**

In a particularly preferred embodiment of the present invention, the at least one further inactivated gene is a gene encoding erythrulose kinase (EYK1). Preferably, the at least one gene encoding erythrulose kinase (Eyk1) of the genetically modified saprotroph comprised the nucleotide sequence of **SEQ ID No. 18** before inactivation, in particular consisted of the nucleotide sequence of **SEQ ID No. 18** before inactivation. Preferably, the erythrulose kinase (Eyk1) encoded by the nucleotide sequence of **SEQ ID No. 18** before inactivation comprises the amino acid sequence of **SEQ ID No. 19**, in particular consists of the amino acid sequence of **SEQ ID No. 19.**

In a preferred embodiment of the present invention, the at least one further inactivated gene is a gene encoding erythritol dehydrogenase (EYD1). Preferably, the at least one gene encoding erythritol dehydrogenase (Eyd1) of the genetically modified saprotroph comprised the nucleotide sequence of **SEQ ID No. 20** before inactivation, in particular consisted of the nucleotide sequence of **SEQ ID No. 20** before inactivation. Preferably, the erythritol dehydrogenase (Eyd1) encoded by the nucleotide sequence of **SEQ ID No. 20** before inactivation comprises the amino acid sequence of **SEQ ID No. 21**, in particular consists of the amino acid sequence of **SEQ ID No. 21.**

In a preferred embodiment of the present invention, the at least one further inactivated gene is a gene encoding erythritol isomerase 1 (EYI1). Preferably, the at least one gene encoding erythritol isomerase 1 (Eyi1) of the genetically modified saprotroph comprised the nucleotide sequence of **SEQ ID No. 22** before inactivation, in particular consisted of the nucleotide sequence of **SEQ ID No. 22** before inactivation. Preferably, the erythritol isomerase 1 (Eyi1) encoded by the nucleotide sequence of **SEQ ID No. 22** before inactivation comprises the amino acid sequence of **SEQ ID No. 23**, in particular consists of the amino acid sequence of **SEQ ID No. 23.**

In another preferred embodiment of the present invention, the at least one further inactivated gene is a gene encoding erythritol isomerase 2 (EYI2). Preferably, the at least one gene encoding isomerase 2 (Eyi2) of the genetically modified saprotroph comprised the nucleotide sequence of **SEQ ID No. 24** before inactivation, in particular consisted of the nucleotide sequence of **SEQ ID No. 24** before inactivation. Preferably, the isomerase 2 (Eyi2) encoded by the nucleotide sequence of **SEQ ID No. 24** before inactivation comprises the amino acid sequence of **SEQ ID No. 25**, in particular consists of the amino acid sequence of **SEQ ID No. 25.**

In a further preferred embodiment of the present invention, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is deleted.

Preferably, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is non-functional.

In a particularly preferred embodiment, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is inactivated by gene knock-out, in particular gene replacement. Preferably, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is inactivated by gene replacement using a deletion cassette.

In a preferred embodiment of the present invention, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is inactivated, in particular made non-functional, by genome editing, in particular by meganucleases, zinc-finger nucleases (ZFNs), transcription activator-like effector nucleases (TALENs) or by the clustered regularly interspaced short palindromic repeats (CRISPR) system.

In a further preferred embodiment of the present invention, the genetically modified saprotroph further comprises at least one gene encoding at least one transketolase. Preferably, the at least one gene encoding at least one transketolase is *tkll,* in particular codon-optimized *tkll.* Preferably, the genetically modified saprotroph further comprises at least one gene encoding *tkll* from *T. reesei,* in particular codon-optimized *tkll* from *T. reesei.* In a preferred embodiment, the at least one gene encoding at least one transketolase comprises the nucleotide sequence of **SEQ ID No. 5**, in particular consists of the nucleotide sequence of **SEQ ID No. 5.** Preferably, the at least one transketolase comprises the amino acid sequence of **SEQ ID No. 6**, in particular consists of the amino acid sequence of **SEQ ID No. 6.**

In a preferred embodiment of the present invention, the genetically modified saprotroph further comprises at least one gene encoding at least one transaldolase. Preferably, the at least one gene encoding at least one transaldolase is *tall,* in particular codon-optimized *tall.* Preferably, the genetically modified saprotroph further comprises at least one gene encoding *tall* from *T. reesei,* in particular codon-optimized *tall* from *T. reesei.* In a preferred embodiment, the at least one gene encoding at least one transaldolase comprises the nucleotide sequence of **SEQ ID No. 7**, in particular consist of the nucleotide sequence of **SEQ ID No. 7.** Preferably, the at least one transaldolase comprises the amino acid sequence of **SEQ ID No. 8**, in particular consists of the amino acid sequence of **SEQ ID No. 8.**

In a further preferred embodiment of the present invention, the genetically modified saprotroph further comprises at least one gene encoding at least one erythritol utilization factor (EUF). Preferably, the at least one gene encoding at least one erythritol utilization factor, is *eufl,* in particular codon-optimized *eufl.* Preferably, the genetically modified saprotroph further comprises at least one gene encoding *eufl* from *T. reesei,* in particular codon-optimized *eufl* from *T. reesei.*

In a preferred embodiment, the at least one gene encoding at least one erythritol utilization factor (EUF) comprises the nucleotide sequence of **SEQ ID No. 16**, in particular consist of the nucleotide sequence of **SEQ ID No. 16.** Preferably, the at least one erythritol utilization factor (EUF) comprises the amino acid sequence of **SEQ ID No. 17**, in particular consists of the amino acid sequence of **SEQ ID No. 17.**

In a preferred embodiment of the present invention, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) of the genetically modified saprotroph is an exogenous polynucleotide sequence.

Preferably, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) of the genetically modified saprotroph is an endogenous polynucleotide sequence.

In a further preferred embodiment, the genetically modified saprotroph according to the present invention comprises at least one exogenous gene encoding at least one membrane-bound alditol transporter. Preferably, genetically modified saprotroph according to the present invention comprises at least one endogenous gene encoding at least one membrane-bound alditol transporter.

Preferably, the genetically modified saprotroph according to the present invention comprises at least one exogenous gene encoding at least one erythrose reductase. In another preferred embodiment, the genetically modified saprotroph according to the present invention comprises at least one endogenous gene encoding at least one erythrose reductase.

Further preferred, the genetically modified saprotroph according to the present invention comprises at least one exogenous gene encoding at least one transketolase. In a preferred embodiment of the present invention, the genetically modified saprotroph according to the present invention comprises at least one endogenous gene encoding at least one transketolase.

Preferably, the genetically modified saprotroph according to the present invention comprises at least one exogenous gene encoding at least one transaldolase. Preferably, the genetically modified saprotroph according to the present invention comprises at least one endogenous gene encoding at least one transaldolase.

Preferably, the genetically modified saprotroph according to the present invention comprises at least one exogenous gene encoding at least one erythritol utilization factor (EUF). Preferably, the genetically modified saprotroph according to the present invention comprises at least one endogenous gene encoding at least one erythritol utilization factor (EUF).

In a preferred embodiment of the present invention, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) is stably or transiently introduced into the genome of the genetically modified saprotroph.

In a particularly preferred embodiment of the present invention, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF), is overexpressed.

Preferably, the at least one gene encoding at least one membrane-bound alditol transporter, in particular the at least one exogenous or endogenous gene encoding at least one membrane-bound alditol transporter, is overexpressed. In a further preferred embodiment, the at least one gene encoding at least one erythrose reductase, in particular the at least one exogenous or endogenous gene encoding at least one erythrose reductase, is overexpressed. Preferably, the at least one gene encoding at least one transketolase, in particular the at least one exogenous or endogenous gene encoding at least one transketolase, is overexpressed. Further preferred, the at least one gene encoding at least one transaldolase, in particular the at least one exogenous or endogenous gene encoding at least one transaldolase, is overexpressed. Preferably, the at least one gene encoding at least one erythritol utilization factor (EUF), in particular the at least one exogenous or endogenous gene encoding at least one erythritol utilization factor (EUF), is overexpressed.

In a further preferred embodiment of the present invention, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) is under the control of a constitutive or inducible promoter. In a preferred embodiment, the constitutive or inducible promoter is a naturally occurring promoter. In a further preferred embodiment, the constitutive or inducible promoter is a synthetic promoter. Preferably, the constitutive promoter is selected from *pki, tef, gpd.* The inducible promoter is preferably selected from *bga1, bxll, cbhl, cbh2, xyn1, xyn2.* Preferably, the at least one gene encoding at least one membrane-bound alditol transporter is under the control of a promoter selected *from pki, tef*, *gpd,* preferably under control of *a pki* promoter. Preferably, the at least one gene encoding at least one erythrose reductase is under the control of a promoter selected from *pki, tef*, *gpd,* preferably under control of a *tef* promoter. Preferably, the at least one gene encoding at least one transketolase is under the control of a promoter selected *from pki, tef*, *gpd,* preferably under control of a *tef*promoter. Preferably, the at least one gene encoding at least one transaldolase is under the control of a promoter selected from *pki, tef*, *gpd,* preferably under control of a *tef* promoter. Preferably, the at least one gene encoding at least one erythritol utilization factor (EUF) is under the control of a promoter selected *from pki, tef*, *gpd,* preferably under control of a *tef* promoter.

In a further preferred embodiment of the present invention, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) is present on a plasmid. Preferably, each of the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) is present on a separate plasmid.

In a particularly preferred embodiment of the present invention, the genetically modified saprotroph is the *Trichoderma reesei* strain deposited at the Westerdijk Fungal Biodiversity Institute under CBS number 146708.

The present invention further pertains to a method for the production of erythritol, comprising the steps:
a) providing at least one genetically modified saprotroph according to the present invention,
b) culturing the at least one genetically modified saprotroph provided in step a) in the presence of a culture medium, so as to obtain erythritol in the culture medium,
c) recovering erythritol from the culture medium.

In a preferred embodiment of the present invention, the culture medium comprises lignocellulosic biomass, in particular straw, and/or at least one residue of dairy product production, in particular whey. Particularly preferred, the culture medium comprises lignocellulosic biomass, in particular straw, preferably hydrolysed straw. According to this particular embodiment, the genetically modified saprotroph is able to grow on lignocellulosic biomass as carbon source, in particular on lignocellulosic biomass as the sole carbon source. In a further preferred embodiment of the present invention, the culture medium comprises at least one residue of dairy product production. Particularly preferred, the culture medium comprises whey. In this way, the method according to the present invention advantageously utilizes inexpensive, abundant and renewable substrates as educts for the production of erythritol.

In a preferred embodiment of the present invention, the culture medium comprises ammonium as a nitrogen source. In a further preferred embodiment of the present invention, the culture medium comprises nitrate as a nitrogen source.

In a further preferred embodiment of the present invention, the culture medium comprises glycerol.

In a preferred embodiment of the present invention, the at least one genetically modified saprotroph provided in step a) is cultured in step b) at a temperature of 25 to 35 °C, preferably 26 to 34 °C, preferably 27 to 33 °C, preferably 28 to 32 °C, preferably 29 to 31 °C, preferably 30 °C.

Preferably, the pH of the culture medium in step b) is within the range of 3 to 8, preferably 3 to 7, preferably 3.5 to 6.5, preferably 3.5 to 6, preferably 4 to 5.5, preferably 4 to 5.

In a particularly preferred embodiment of the present invention, step b) is conducted until a concentration of erythritol in the culture medium of at least 1 g/L, preferably at least 1.5 g/L, preferably at least 2 g/L, preferably at least 2.5 g/L, preferably at least 3 g/L, is reached.

In a preferred embodiment of the present invention, in step b) at least one substrate that causes osmotic stress is added to the culture medium. Preferably, the at least one substrate that causes osmotic stress is added to the culture medium after 10 hours of cultivation, preferably 12 hours of cultivation, preferably 14 hours of cultivation, preferably 16 hours of cultivation.

In a further preferred embodiment of the present invention, the substrate that causes osmotic stress is selected from glycerol and sodium chloride or a combination thereof. Preferably, the substrate that causes osmotic stress is glycerol. Preferably, the substrate that causes osmotic stress is sodium chloride.

In a preferred embodiment of the present invention, in step c) erythritol is recovered by crystallisation. Particularly preferred, the recovery of erythritol from the culture medium in step c) comprises the steps:
i) removal of biomass, preferably removal of biomass by filtration,
ii) decolorisation of the culture medium, preferably decolorisation of the culture medium with active carbon,
iii) desalting the culture medium, preferably desalting and decolorizing the culture medium with at least one ion exchange resin, and
iv) concentrating and crystallising the erythritol.

The present invention also pertains to the use of a genetically modified saprotroph according to the present invention for the production of erythritol.

The term "genetically modified" and its grammatical equivalents as used herein refer to one or more alterations of a nucleic acid, e.g. the nucleic acid within the genome of an organism, in particular within the genome of a saprotroph. A "genetically modified" saprotroph can refer to a saprotroph with an added, deleted and/or altered, in particular inactivated, gene.

In the context of the present invention, the term "erythrose reductase" pertains to any enzyme that catalyses reversibly the reduction of an aldose to an alditol, wherein the enzyme has predominant specific activity for the reduction of erythrose to erythritol. Thus, an "erythrose reductase" according to the present invention exhibits a high specificity and affinity for the substrate erythrose. Particularly, the "erythrose reductase" has a higher specificity for erythrose than for any other substrate, such as glycerol.

The term "membrane-bound alditol transporter" as used in the context of the present invention designates a membrane protein suitable to reversibly transport alditols across the outer membrane, in particular to transport intracellularly produced alditols to the culture medium. According to the present invention, a "membrane-bound alditol transporter" is suitable to transport intracellularly produced erythritol across the membrane to the culture medium.

In the context of the present invention, the term "transketolase" refers to an enzyme of the pentose phosphate pathway catalysing the thiamine-dependent reversible transfer of a C-2 unit from a ketose donor to an acceptor aldopentose. In particular, the "transketolase" catalyses the transfer of a C-2 unit from xylulose-5-phosphate to ribose-5-phosphate forming seduheptulose-7-phosphate and glyceraldehyde-3-phosphate.

The term "transaldolase" designates an enzyme of the non-oxidative branch of the pentose phosphate pathway catalysing the reversible transfer of a C-3 unit from a ketose donor to an acceptor aldose. In particular, the "transketolase" catalyses the transfer of a C-3 unit from sedoheptulose-7-phosphate to glyceraldehyde-3-phosphate forming erythrose-4-phosphate and fructose-6-phosphate.

In the context of the present invention an "inactivated" gene is a gene which can temporarily or permanently not be transcribed or can be transcribed but the transcript is not accessible for gene translation. According to the present invention, the inactivation of a gene includes but is not limited to inactivation by partial or complete gene deletion, partial or complete gene replacement, gene repression, gene insertion, and gene mutation. "Inactivation" in the context of the present invention further includes any post-transcriptional gene regulation, in particular by RNAi or siRNA, resulting in inhibition of translation of the transcript into the gene product.

In the context of the present invention a "non-functional" gene is a gene that is present in the genome of an organism or a plasmid in an organism which gene is either not transcribed or is transcribed but the transcript is not translated to the gene product.

In the context of the present invention a "deleted" gene is a gene that has previously been present in the genome of an organism or a plasmid in an organism but has been completely removed from the genome of the organism or the plasmid in the organism.

In the context of the present invention the verb "to overexpress" refers to the artificial expression of a gene in increased quantity. This includes the inducible overexpression of a gene which can be regulated using an inducible promoter and the constant overexpression of a gene using a constitutive promoter.

In the context of the present invention, the term "a" is meant to include the meaning of "one" or "one or more".

In the context of the present invention, the term "comprising" preferably has the meaning of "containing" or "including", meaning including the specifically identified elements without excluding the presence of further elements. However, in a preferred embodiment, the term "comprising" is also understood to have the meaning of "consisting essentially of' and in a further preferred embodiment the meaning of "consisting of'.

The terms "and/or" is used herein to express the disclosure of any combination of individual elements connected within a listing. Solely for illustrative purposes, the expression "A, B, and/or C" can mean A individually, B individually, C individually, (A and B), (B and C), (A and C), and (A, B and C).

Further preferred embodiments of the invention are subject of the subclaims.

The invention is further described by way of the following example and the accompanying figures.
Fig. 1 shows a comparison dry biomass of a *T. reesei* wild-type strain (WT), of a *T. reesei* strain (strain 1) overexpressing the alditol transporter gene *fpsl* and comprising an inactivated gene encoding mannitol 1-phosphate 5-dehydrogenase (*mpdh*) and of a *T. reesei* strain (strain 2) overexpressing the alditol transporter gene *fps1* and the gene *err1* encoding erythrose reductase as well as comprising an inactivated gene encoding mannitol 1-phosphate 5-dehydrogenase (*mpdh*) after 40 hours of cultivation.
Fig. 2 shows the concentration of glucose in the culture medium after 18, 28, 30, 32, 34, 36, 38 and 40 hours of cultivation of the wild-type strain (WT) and strains 1 and 2.
Fig. 3 shows the concentration of glycerol in the culture medium after 18, 28, 30, 32, 34, 36, 38 and 40 hours of cultivation of the wild-type strain (WT) and strains 1 and 2.
Fig 4 shows the concentration of erythritol in the culture medium after 18, 28, 30, 32, 34, 36, 38 and 40 hours of cultivation of the wild-type strain (WT) and strains 1 and 2.
Fig. 5 shows a plasmid map of pKR_ReAsl1fps1 (see also SEQ ID No. 9).
Fig. 6 shows a plasmid map of pKR_Rpyr4err1 (see also SEQ ID No .10).
Fig. 7 shows a deletion cassette for deletion *of mpdh* (see also SEQ ID No. 11).

### Example

### 1.1 Comparison of T. reesei strains

For the characterization of the phenotype the following strains were used:
- the wild type strain QM6aΔ*tmus53*, short-termed "WT",
- QM6aΔ*tmus53fps1*(Re*asl1*)Δ*mpdh*(hygR), short-termed "strain 1", and
- QM6aΔ*tmus53fps1*(Re*asl1*)OEerr1(Re*pyr4*)Δ*mpdh*(hygR), short-termed "strain 2". This strain has been deposited on 25.05.2020 in the Restricted CBS collection of the Westerdijk Fungal Biodiversity Institute, Utrecht, The Netherlands by the depositor Technische Universität Wien (Gumpendorfer Straße 1a, 1060 Vienna, Austria) under the stipulations of the Budapest Treaty on the International Recognition of the Deposit of Microorganisms for the Purpose of Patent Procedure. The corresponding viability certificate has been emitted and the strain has been given accession number CBS 146708.

### 1.2 Materials and Methods

### 1.2.1 Generation of T. reesei strains

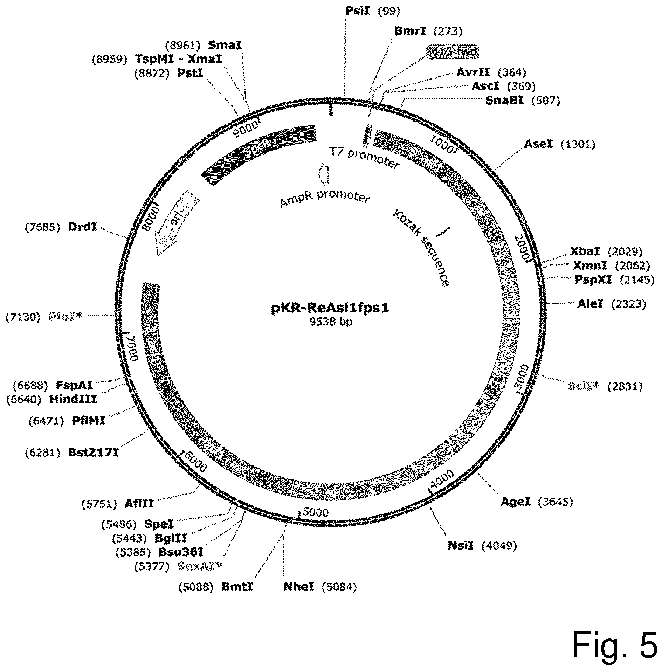

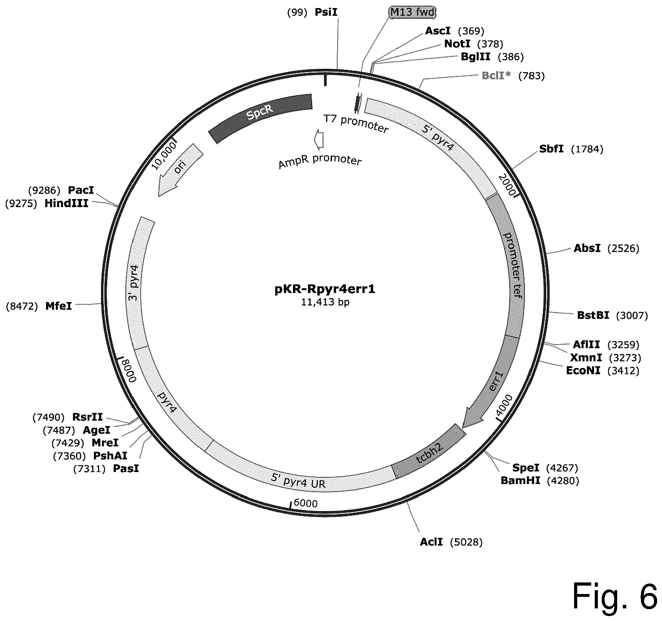

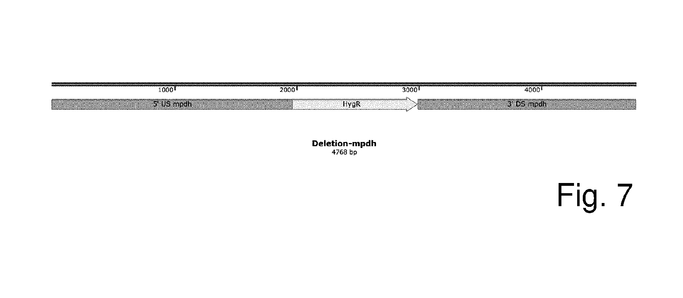

The parental *T. reesei* strain used in the experiments is auxotroph for uridine (lack of functional *pyr4),* arginine (lack of functional *asl1*), histidine (lack of functional 80820 HisG) and is further resistant to hygromycin (hygR). The first transformation with the plasmid pKR_Reasl1fps1 (see Fig. 5) to restore the *asl1* locus not only introduces the *fps1* gene into the genome, but also makes the strain hygromycin sensible again. The *err1* gene encoding erythrose reductase was introduced using the plasmid pKR_Repyr4err1 (see Fig. 6). The deletion of *mpdh* was done by transformation of a deletion cassette (see Fig. 7) to regain hygromycin resistance by homologous recombination. All transformations are performed as protoplast transformations. Typically, 30 to 50 µg of digested plasmid DNA (in 15 µl sterile double-distilled water) were used for transformation of 10⁷ protoplasts (in 200 µl). For selection for resistance against hygromycin B, 100 µl to 2 ml of the transformation reaction mixture was added to 10 ml melted, 50 °C warm malt extract (MEX) agar containing 1.2 M sorbitol. This mixture was poured into sterile petri dishes and incubated at 30 °C for 5 h after solidification. Subsequently, 10 ml melted, 50 °C MEX agar containing 1.2 M sorbitol and a double concentration of hygromycin B was poured on top of the protoplast-containing layer.

Plates were incubated at 30 °C for 2 to 5 days until colonies were visible. For selection for prototrophy, 100 µl to 2 ml of the transformation reaction mixture was added to 20 ml melted, 50 °C warm minimal medium agar containing 1.2 M sorbitol and, in the case of *asl1* back transformation, additionally 0.25 mM L-arginine. This mixture was poured into sterile petri dishes and after solidification was incubated at 30 °C for 3 to 7 days until colonies were visible.

### 1.2.2 Cultivation of T. reesei and induction of erythritol synthesis

In the experiment ammonium was used as nitrogen source and glucose as carbon source under osmotic stress. The total duration of cultivation was 40 hours. After 10 hours of cultivation for biomass production, sodium chloride was added to the medium in order to initiate osmotic stress. Culture broth samples have been taken at the time of this addition and next after 28 hours of total cultivation time and then every two hours until end of the cultivation. Samples were centrifuged to separate the mycelium from the supernatant.

Cultivation was performed with the wild-type strain (WT), QM6a_fm (strain 1) and QM6a_fem (strain 2) in 100 ml shake flasks containing 25 ml cultivation medium ((NH₄)₂SO₄ 2.80 g/l, KH₂PO₄ 4.00 g/l, MgSO₄x7H₂O 1.0 g/l, NaCl 0.5 g/l, peptone from Casein 0.1 g/l, Tween^{®}80 0.5 g/1), supplemented with 1.5 ml/l trace element solution (FeSO₄x7H₂O 0.90 mM, MnSO₄xH₂O 0.50 mM, ZnSO₄x7H₂O 0.24 mM, CaCl₂x7H₂O 0.68 mM). For inoculation 10⁹ conidiospores per liter were used. To all flasks 0.3 ml of 0.2 M histidine and 0.25 ml of 0.2 M uridine was added. After 10 hours 0.5 ml of 5 M NaCl was added. The agitation rate was 180 rpm, the temperature was 30 °C.

After the cultivation the whole cultivation broth was filtered through miracloth, the mycelia were pressed dry with Whatman filter paper and dried at 80 °C overnight.

Glucose, glycerol, mannitol and erythritol concentrations were quantified by high-performance liquid chromatography (HPLC) (Shimadzu Prominence Series equipped with a RID-20A refractive-index detector). Culture supernatants were filtered through 0.20 µm membranes and measured by HPLC using a Rezex RCM-Monosaccharide column (300 mm x 7.8 mm; Phenomenex, Torrance, CA, USA). The mobile phase was 30% acetonitrile (HPLC grade). For sample analysis, the column was eluted at 60 °C with 30% acetonitrile (HPLC grade) at a flow rate of 0.6 ml/min.

### 1.3 Results

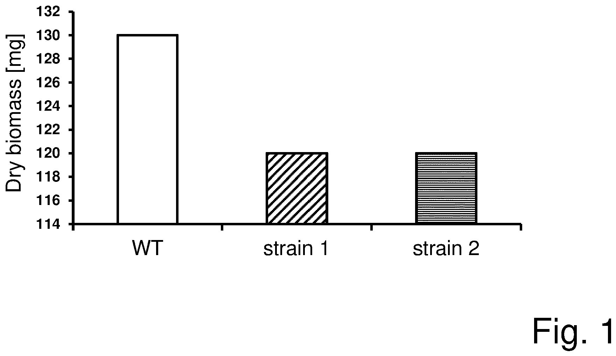

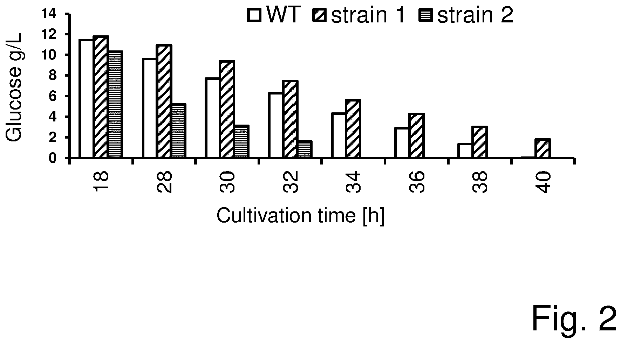

The biomass production at the end of the cultivation is nearly the same for all strains. WT produces slightly more biomass (Fig. 1). The glucose consumption is faster in strain 2 compared to WT and strain 1, which is deduced from concentration measurements in the respective supernatants (Fig. 2).

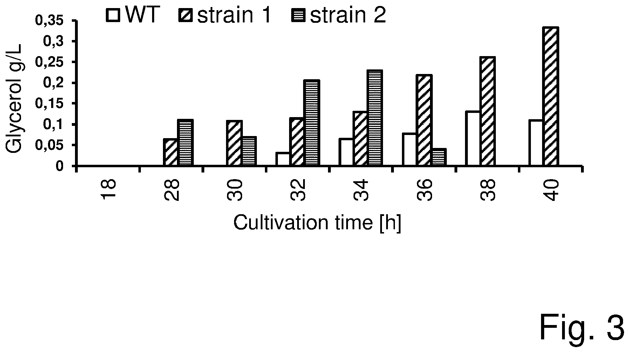

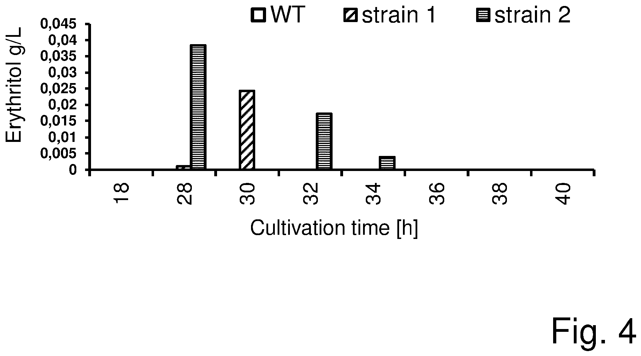

The glycerol concentration in the supernatant of strain 1 is always higher than in the WT due to the deletion of mannitol 1-phosphate 5-dehydrogenase (Fig. 3) since this deletion results in elimination of the production of mannitol and the strains produce more of the osmolyte glycerol. Whereas strain 1 shows a constant increase in glycerol production, strain 2 bearing the overexpressed *err1* gene shows a fluctuating accumulation of glycerol over time (Fig. 3). Erythritol is only detectable in the supernatants of strains bearing the transporter FPS1 of *Saccharomyces cerevisiae* (Fig. 4). Investigations on the consumption of erythritol demonstrated that all strains, including WT, can grow on erythritol as sole carbon source (data not shown). The presence of erythritol in the supernatant seems to coincide with the secretion of glycerol and it is higher for strain 2 (Fig. 3 and Fig. 4).

## Claims

1. A genetically modified saprotroph comprising at least one gene encoding at least one membrane-bound alditol transporter, at least one gene encoding at least one erythrose reductase and at least one inactivated gene encoding mannitol 1-phosphate 5-dehydrogenase, wherein the saprotroph is a filamentous fungus selected from the genera *Hypocrea*, *Gibberella, Aspergillus* and *Penicillium.*

2. The genetically modified saprotroph according to claim 1, wherein the saprotroph is a filamentous fungus selected from *Hypocrea jecorina (Trichoderma reesei), Gibberella zeae, Aspergillus niger, Aspergillus nidulans, Aspergillus oryzae* and *Penicillium chrysogenum.*

3. The genetically modified saprotroph according to claim 1 or 2, wherein the saprotroph is *Hypocrea jecorina (Trichoderma reesei).*

4. The genetically modified saprotroph according to any one of the preceding claims, wherein the at least one gene encoding at least one membrane-bound alditol transporter is *fps1* from *Saccharomyces cerevisiae.*

5. The genetically modified saprotroph according to any one of the preceding claims, wherein the at least one gene encoding at least one erythrose reductase is *err1* from *Trichoderma reesei, Aspergillus niger,* or *Fusarium graminearum.*

6. The genetically modified saprotroph according to any one of the preceding claims, wherein the saprotroph further comprises at least one gene encoding at least one transketolase, in particular *tkll* from *Hypocreajecorina (T. reesei).*

7. The genetically modified saprotroph according to any one of the preceding claims, wherein the saprotroph further comprises at least one gene encoding at least one transaldolase, in particular *tall* from *Hypocreajecorina (T. reesei).*

8. The genetically modified saprotroph according to any one of the preceding claims, wherein the saprotroph further comprises at least one inactivated gene encoding phospho-2-dehydro-3-deoxyheptonate aldolase 1 (Dhaps-1).

9. The genetically modified saprotroph according to any one of the preceding claims, wherein the at least one gene encoding at least one membrane-bound alditol transporter, the at least one gene encoding at least one erythrose reductase, the at least one gene encoding at least one transketolase and/or the at least one gene encoding at least one transaldolase is overexpressed.

10. A method for the production of erythritol, comprising the steps:
a) providing at least one genetically modified saprotroph according to any one of claims 1 to 9,
b) culturing the at least one genetically modified saprotroph provided in step a) in the presence a culture medium, so as to obtain erythritol in the culture medium,
c) recovering erythritol from the culture medium.

11. The method according to claim 10, wherein the culture medium comprises lignocellulosic biomass, in particular straw, and/or at least one residue of dairy product production, in particular whey.

12. The method according to claim 10 or 11, wherein the culture medium comprises nitrate as a nitrogen source.

13. The method according to claims 10 to 12, wherein the culture medium comprises glycerol.

14. The method according to claims 10 to 13, wherein step b) is conducted until a concentration of erythritol in the culture medium of at least 1 g/L, preferably at least 1.5 g/L, preferably at least 2 g/L, preferably at least 2.5 g/L, preferably at least 3 g/L, is reached.

15. The method according to claim 10 or 14, wherein in step b) at least one substrate that causes osmotic stress is added to the culture medium after 14 hours of cultivation.

16. The method according to claim 15, wherein the substrate that causes osmotic stress is glycerol and/or sodium chloride.

17. Use of a genetically modified saprotroph according to any one of claims 1 to 9 for the production of erythritol.

## Patentansprüche

1. Gentechnisch veränderter Saprotroph, der mindestens ein mindestens einen membrangebundenen Alditol-Transporter kodierendes Gen, mindestens ein mindestens eine Erythrose-Reduktase kodierendes Gen, und mindestens ein Mannitol-1-Phosphat-5-Dehydrogenase kodierendes inaktiviertes Gen, umfasst, wobei der Saprotroph ein filamentöser Pilz ausgewählt aus den Gattungen *Hypocrea, Gibberella, Aspergillus* und *Penicillium* ist.

2. Gentechnisch veränderter Saprotroph nach Anspruch 1, wobei der Saprotroph ein filamentöser Pilz ausgewählt aus *Hypocrea jecorina (Trichoderma reesei), Gibberella zeae, Aspergillus niger, Aspergillus nidulans, Aspergillus oryzae* und *Penicillium chrysogenum* ist.

3. Gentechnisch veränderter Saprotroph nach Anspruch 1 oder 2, wobei der Saprotroph *Hypocrea jecorina (Trichoderma reesei)* ist.

4. Gentechnisch veränderter Saprotroph nach einem der vorhergehenden Ansprüche, wobei das mindestens eine mindestens einen membrangebundenen Alditol-Transporter kodierende Gen, *fps1* aus *Saccharomyces cerevisiae* ist.

5. Gentechnisch veränderter Saprotroph nach einem der vorhergehenden Ansprüche, wobei das mindestens eine mindestens eine Erythrose-Reduktase kodierende Gen, *err1* aus *Trichoderma reesei, Aspergillus niger* oder *Fusarium graminearum* ist.

6. Gentechnisch veränderter Saprotroph nach einem der vorhergehenden Ansprüche, wobei der Saprotroph ferner mindestens ein mindestens eine Transketolase kodierendes Gen, insbesondere *tkl1* aus *Hypocrea jecorina (T. reesei),* umfasst.

7. Gentechnisch veränderter Saprotroph nach einem der vorhergehenden Ansprüche, wobei der Saprotroph ferner mindestens ein mindestens eine Transaldolase kodierendes Gen, insbesondere *tal1* aus *Hypocrea jecorina (T. reesei),* umfasst.

8. Gentechnisch veränderter Saprotroph nach einem der vorhergehenden Ansprüche, wobei der Saprotroph ferner mindestens ein Phospho-2-dehydro-3-deoxyheptonat-Aldolase 1 (Dhaps-1) kodierendes inaktiviertes Gen umfasst.

9. Gentechnisch veränderter Saprotroph nach einem der vorhergehenden Ansprüche, wobei das mindestens eine mindestens einen membrangebundenen Alditol-Transporter kodierende Gen, das mindestens eine mindestens eine Erythrose-Reduktase kodierende Gen, das mindestens eine mindestens eine Transketolase kodierende Gen, und/oder das mindestens eine mindestens eine Transaldolase kodierende Gen, überexprimiert wird.

10. Verfahren zur Herstellung von Erythritol umfassend die Schritte:
a) Bereitstellen mindestens eines gentechnisch veränderten Saprotrophen nach einem der Ansprüche 1 bis 9,
b) Kultivieren des mindestens einen in Schritt a) bereitgestellten gentechnisch veränderten Saprotrophen in Gegenwart eines Kulturmediums zum Erhalt von Erythritol in dem Kulturmedium,
c) Gewinnen von Erythritol aus dem Kulturmedium.

11. Verfahren nach Anspruch 10, wobei das Kulturmedium Lignozellulose-haltige Biomasse, insbesondere Stroh, und/oder mindestens einen Rückstand aus der Milchproduktherstellung, insbesondere Molke, umfasst.

12. Verfahren nach Anspruch 10 oder 11, wobei das Kulturmedium Nitrat als Stickstoffquelle umfasst.

13. Verfahren nach einem der Ansprüche 10 bis 12, wobei das Kulturmedium Glycerin umfasst.

14. Verfahren nach einem der Ansprüche 10 bis 13, wobei Schritt b) durchgeführt wird, bis eine Konzentration von Erythritol im Kulturmedium von mindestens 1 g/L, vorzugsweise mindestens 1,5 g/L, vorzugsweise mindestens 2 g/L, vorzugsweise mindestens 2,5 g/L, vorzugsweise mindestens 3 g/L, erreicht ist.

15. Verfahren nach Anspruch 10 oder 14, wobei in Schritt b) mindestens ein osmotischen Stress verursachendes Substrat dem Kulturmedium nach 14 Stunden Kultivieren hinzugefügt wird.

16. Verfahren nach Anspruch 15, wobei das osmotischen Stress verursachende Substrat, Glycerin und/oder Natriumchlorid ist.

17. Verwendung eines gentechnisch veränderten Saprotrophen nach einem der Ansprüche 1 bis 9 zur Herstellung von Erythritol.

## Revendications

1. Un saprotrophe génétiquement modifié comprenant au moins un gène codant pour au moins un transporteur d'alditol lié à la membrane, au moins un gène codant pour au moins une érythrose réductase et au moins un gène inactivé codant pour la mannitol 1-phosphate 5-déhydrogénase, dans lequel le saprotrophe est un champignon filamenteux choisi parmi les genres *Hypocrea, Gibberella, Aspergillus* et *Penicillium.*

2. Le saprotrophe génétiquement modifié selon la revendication 1, dans lequel le saprotrophe est un champignon filamenteux choisi parmi *Hypocrea jecorina (Trichoderma reesei), Gibberella zeae, Aspergillus niger, Aspergillus nidulans, Aspergillus oryzae* et *Penicillium chrysogenum.*

3. Le saprotrophe génétiquement modifié selon la revendication 1 ou 2, dans lequel le saprotrophe est *Hypocrea jecorina (Trichoderma reesei).*

4. Le saprotrophe génétiquement modifié selon l'une quelconque des revendications précédentes, dans lequel le au moins un gène codant pour au moins un transporteur d'alditol lié à la membrane est *fps1* de *Saccharomyces cerevisiae.*

5. Le saprotrophe génétiquement modifié selon l'une quelconque des revendications précédentes, dans lequel le au moins un gène codant pour au moins une érythrose réductase est *err1* de *Trichoderma reesei, Aspergillus niger,* ou *Fusarium graminearum.*

6. Le saprotrophe génétiquement modifié selon l'une quelconque des revendications précédentes, dans lequel le saprotrophe comprend en outre au moins un gène codant pour au moins une transcétolase, particulièrement *tkl1* de *Hypocrea jecorina (T. reesei).*

7. Le saprotrophe génétiquement modifié selon l'une quelconque des revendications précédentes, dans lequel le saprotrophe comprend en outre au moins un gène codant pour au moins une transaldolase, particulièrement *tal1* de *Hypocrea jecorina (T. reesei).*

8. Le saprotrophe génétiquement modifié selon l'une quelconque des revendications précédentes, dans lequel le saprotrophe comprend en outre au moins un gène inactivé codant pour la phospho-2-déhydro-3-déoxyheptonate aldolase 1 (Dhaps-1).

9. Le saprotrophe génétiquement modifié selon l'une quelconque des revendications précédentes, dans lequel le au moins un gène codant pour au moins un transporteur d'alditol lié à la membrane, le au moins un gène codant pour au moins une érythrose réductase, le au moins un gène codant pour au moins une transcétolase et/ou le au moins un gène codant pour au moins une transaldolase est surexprimé.

10. Un procédé de production d'érythritol, comprenant les étapes suivantes
a) fournir au moins un saprotrophe génétiquement modifié selon l'une quelconque des revendications 1 à 9,
b) cultiver le au moins un saprotrophe génétiquement modifié fourni à l'étape a) en présence d'un milieu de culture, de façon à obtenir de l'érythritol dans le milieu de culture,
c) récupérer l'érythritol à partir du milieu de culture.

11. Le procédé selon la revendication 10, dans lequel le milieu de culture comprend de la biomasse lignocellulosique, particulièrement de la paille, et/ou au moins un résidu de production de produits laitiers, particulièrement du lactosérum.

12. Le procédé selon la revendication 10 ou 11, dans lequel le milieu de culture comprend du nitrate comme source d'azote.

13. Le procédé selon les revendications 10 à 12, dans lequel le milieu de culture comprend du glycérol.

14. Le procédé selon les revendications 10 à 13, dans lequel l'étape b) est conduite jusqu'à atteindre une concentration en érythritol dans le milieu de culture d'au moins 1 g/L, de préférence d'au moins 1,5 g/L, de préférence d'au moins 2 g/L, de préférence d'au moins 2,5 g/L, de préférence d'au moins 3 g/L.

15. Le procédé selon la revendication 10 ou 14, dans lequel, à l'étape b), au moins un substrat qui provoque un stress osmotique est ajouté au milieu de culture après 14 heures de culture.

16. Le procédé selon la revendication 15, dans lequel le substrat qui provoque un stress osmotique est le glycérol et/ou le chlorure de sodium.

17. Une utilisation d'un saprotrophe génétiquement modifié selon l'une quelconque des revendications 1 à 9 pour la production d'érythritol.
